(19)

Europäisches Patentamt
European Patent Office
Office européen des brevets

(11) **EP 4 245 839 A1**

(12) **EUROPEAN PATENT APPLICATION**

(43) Date of publication:
**20.09.2023 Bulletin 2023/38**

(21) Application number: **22305292.9**

(22) Date of filing: **14.03.2022**

(51) International Patent Classification (IPC):
*C12M 1/34* (2006.01)     *C12M 1/00* (2006.01)

(52) Cooperative Patent Classification (CPC):
**C12M 41/00; C12M 23/26; C12M 29/26;
C12M 41/32**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Fluigent
94270 Le Kremlin-Bicêtre (FR)**
• **SORBONNE UNIVERSITE
75006 Paris (FR)**
• **Centre national de la recherche scientifique
75016 Paris (FR)**
• **Institut Curie
75005 Paris (FR)**
• **INSTITUT NATIONAL DE LA SANTE ET
DE LA RECHERCHE MEDICALE (INSERM)
75013 Paris (FR)**

(72) Inventors:
• **BOUQUEREL, Charlotte
Lyon (FR)**
• **CESAR, William
Paris (FR)**
• **VIOVY, Jean-Louis
Paris (FR)**
• **GENTRIC, Géraldine
Melun (FR)**
• **DESCROIX, Stéphanie
Paris (FR)**
• **PARRINI, Maria-Carla
Paris (FR)**
• **MECHTA-GRIGORIOU, Fatima
Paris (FR)**

(74) Representative: **Bandpay & Greuter
30, rue Notre-Dame des Victoires
75002 Paris (FR)**

(54) **METHOD FOR MONITORING AT LEAST ONE SUBSTANCE PRODUCED OR CONSUMED BY A LIVING ENTITY**

(57)     The invention relates to a method for monitoring at least one substance which may be produced or consumed by at least one living entity, and to an assembly for implementing the method. The method comprises flowing liquid medium having a controlled concentration of a dissolved gas and a controlled flow rate to a culture system (201) and taking at least one measurement within the culture system (201) so as to determine the presence, concentration or amount of the at least one substance in the liquid medium (205) in the culture system (201); and/or taking at least a first measurement upstream of the culture system (201) and a second measurement downstream of the culture system (201), and determining a concentration or amount of the substance consumed or produced by the at least one living entity based on the difference between the second measurement and the first measurement.

FIG. 1

EP 4 245 839 A1

## Description

### Technical field

The present invention relates to a method for monitoring at least one substance which may be produced/secreted or consumed by at least one living entity. The invention also relates to an assembly for implementation of the method. In some of its aspects, it also relates to monitoring living entities in a controlled dissolved gaseous environment.

### Technical background

Cell cultures such as microfluidic cell cultures constitute an important technology for biological research applications including molecular and drug screening or toxicity screening. This technology can provide improved cell microenvironment reproduction, meaning the co-culture of different types of cells, high-quality cell-based data, reduced reagent consumption, at low cost.

By measuring the variations of nutrients, metabolites, organic molecules and biological and molecular elements in *in vitro* samples, one can retrieve information about the bioenergetic state and physiology of cells without adding labels or manipulation. This enables the measurement of analytes moving in and out of cells, which are sensitive indicators of changes in cellular physiology or pathophysiology. It also allows for a non-invasive procedure as the cells can be queried repeatedly over time to generate kinetic data. As a result, the use of this technology has become increasingly valued in the fields of pathophysiology, cancer research, aging, immunology, drug discovery, disease diagnosis and analysis, and a variety of other therapeutic and experimental work.

The manner in which cells remodel bioenergetic pathways can be monitored by adding reagents and measuring the response in real time. The two primary energy-generating pathways of cells are mitochondrial respiration and glycolysis. Methods have been proposed for testing metabolic baseline and potential for each of these two pathways by measuring $O_2$ consumption and pH variations. Instead of stimulating and analyzing the two major energetic pathways individually, both pathways can be stimulated simultaneously, leading to a reduction of the samples used. The protocol generally comprises a step of measuring an initial oxygen consumption rate (OCR) in pmol/min and an initial extracellular acidification rate (ECAR) in mpH/min. A mitochondrial uncoupling agent and an ATP synthase inhibitor are then simultaneously administered to the cell sample. OCR and ECAR are then measured simultaneously, enabling calculation of the metabolic potential of the cell sample.

Document CN107430055 A relates to a method and system for determining integrated metabolic baseline and potential of living cells. Embodiments relate to measuring the activity of each of the two major energy-generating pathways within the cell: mitochondrial respiration and glycolysis, first under baseline conditions, and again after applying a stress to the cells to demand increased energy supply.

However, the implementation of such a method poses a number of challenges.

For example, testing is generally implemented under normoxic (20.9% oxygen concentration, i.e., the oxygen concentration in air) conditions, which, for most healthy tissues is considered a hyperoxic replication of an *in vivo* environment. Results can therefore be misleading, as most tissues in fact experience physioxic (5 to 10% oxygen concentration) or hypoxic (lower concentration than healthy tissue, such as in tumor microenvironment, often below 3% oxygen concentration) conditions. Even when such environments are attempted to be replicated, further challenges are faced in achieving an efficient equilibration time, controlling the gas concentration within the cell culture medium and performing the tests using a single system.

In addition, correction algorithms often need to be employed to overcome error induced by oxygen and other gases diffusing through plastic apparatus into the test sample. This involves additional cumbersome tasks of resetting algorithm parameters, the implementation of secondary programs to reset the parameters, and the adaptation of assay procedures and workflows.

Moreover, in the case of defining altered tumor metabolism, features such as basal respiration, proton leaks, ATP production and non-mitochondrial oxygen consumption are commonly determined individually as opposed to by one single method.

Further, present methods commonly use cell monolayers (2D) for culture and analysis, although 3D culture can affect various parameters such as proliferation, cell morphology, protein and gene expression, and metabolism.

In addition, although acidification rate is measured in mpH/min, various proton sources can affect the pH in addition to the protons' efflux during lactate export.

Also, after the injection of an inhibitor (for example, oligomycin), ATP production is typically determined/deduced from by measuring $O_2$ in mmHg as opposed to measuring the molecular concentration directly. This shortcut in the analysis is an extrapolation.

In addition, present methods generally consider the step of injection and not perfusion, rinsing or sampling and so sequential tests are influenced by the components secreted by the cells in the previous tests, limiting steady state measurements.

Some methods provide solutions to aspects of such challenges.

Document CN110669671 A relates to a cell metabolism continuous monitoring device based on a hollow fiber membrane and method of using same. The cell metabolism continuous monitoring device is a hollow column with a multilayer membrane structure, and sequentially comprises a cell attachment growth layer, the hollow fiber membrane, a basic conductive layer, an electron transfer layer and a biological functional material layer from the outside to the inside. A monitoring method comprises the steps that cells to-be-detected grow and reproduce on the cell attachment growth layer, the concentration of nutrients and metabolites in a culture solution around the cells is changed, the culture solution flows through holes in the hollow fiber membrane, and specific catalytic decomposition of glucose or lactic acid or hydrogen peroxide by a biological functional material layer on the innermost layer is achieved.

Document CN104428672 A relates to a method for determining and/or monitoring at least one condition of a three-dimensional cell culture system comprising at least one growth section, wherein the at least one condition is selected from the group consisting of physiological condition, vitality, and metabolism status. The document further relates to an optical sensor device configured to carry out said method.

Document WO2008024855 A1 relates to systems, methods and devices for the monitoring of the metabolism and the health of cultured cells. The devices simplify the loading of cells and enable loading under sterile conditions. The devices also allow for the culture, measuring and monitoring of the metabolism of a population of cells, and in particular, the accurate measuring of metabolic states simultaneously in multiple cell populations.

Document KR20160149109 A relates to an experimental device for cell culture. The experimental device for cell culture comprises: an X-Y driving stage table; a microplate; a sensor board; a plurality of cartridges; a plurality of drug injectors with fixed positions; and a controller. The microplate comprises a plurality of wells accommodating cells and having a sensor membrane for detecting dissolved oxygen and a sensor membrane for detecting the pH, and is mounted on the X-Y driving stage table. The sensor board is mounted on an upper portion of the X-Y driving stage table, and comprises: a pair of light emitting and receiving elements for detecting the dissolved oxygen, which vertically corresponds to the sensor membrane for detecting the dissolved oxygen by the wells of the microplate mounted on the X-Y driving stage table; and a pair of light emitting and receiving elements for detecting the pH, which vertically corresponds to the sensor membrane for detecting the pH by the wells of the microplate.

Document FR 3075823 discloses a method for analyzing a cell sample in a microfluidic device, wherein cell secretions are measured. This method does not allow to successively feed liquid media having different oxygen concentrations.

Within this context, there is a need to provide an improved method for the monitoring of at least one substance, which may be produced or consumed by cells or other living entities, that is more efficient and more versatile than state of the art methods, regarding notably, but not exclusively, the control of dissolved gas environment, and the timescale of such control.

## Summary of the invention

It is therefore an object of this invention to provide a method for monitoring at least one substance which may be produced or consumed by at least one living entity, the method comprising:

- providing a culture system comprising said living entity;
- providing a feeding device fluidically connected to the culture system, the feeding device comprising at least one feeding module, and each feeding module comprising:

  - a liquid reservoir containing a liquid medium and provided with a gas inlet, a gas outlet and a liquid outlet,
  - an admission line configured to provide a gaseous medium into the liquid reservoir, the admission line being connected to the gas inlet, and
  - an emission line configured to withdraw the gaseous medium from the liquid reservoir, the emission line being connected to the gas outlet;

  the feeding device further comprising at least one control valve associated with the admission line(s) and at least one control valve associated with the emission line(s);
- flowing gaseous medium into the at least one liquid reservoir via the admission line and out of the at least one liquid reservoir via the emission line, so as to apply a pressure in the at least one liquid reservoir;
- flowing the liquid medium with a controlled concentration of a dissolved gas and at a controlled flow rate, from the at least one liquid reservoir to the culture system due to the pressure in the at least one liquid reservoir;

the method further comprising:

- taking at least one measurement within the culture system so as to determine the presence, concentration or amount of the at least one substance in the liquid medium in the culture system; and/or
- taking at least a first measurement upstream of the culture system and a second measurement downstream of the culture system, and determining a concentration or amount of the substance consumed or produced by the at least one living entity based on the difference between the second measurement and the first measurement.

In some variations, the method comprises taking multiple successive measurements so as to determine the evolution over time of the presence or concentration of the at least one substance.

In some variations, the at least one substance is a nutrient or metabolite, preferably selected from lactate, glucose and/or an amino acid; and, preferably, the measurement comprises the direct measurement of the at least one substance.

In some variations, the feeding device comprises at least two feeding modules, comprising different liquid media, the method comprising a step of flowing liquid medium from the liquid reservoir of a first feeding module to the culture system, and then flowing liquid medium from the liquid reservoir of a second feeding module to the culture system.

In some variations, the method comprises a step of flowing liquid medium in sequence or in parallel from the at least one liquid reservoir to multiple culture systems.

In some variations, the method comprises a step of collecting liquid medium from the culture system and refilling at least one liquid reservoir of the feeding device with the collected liquid medium.

In some variations, the method comprises a step of controlling the control valve(s) associated with the admission line(s) and/or the control valve(s) associated with the emission line(s) to adjust at least one concentration of dissolved gas, preferably oxygen and/or carbon dioxide, in the liquid medium to a predetermined value, preferably using a closed loop regulation and/or the method comprising a step of controlling the control valve(s) associated with the admission line(s) and/or the control valve(s) associated with the emission line(s) to adjust the pH of the liquid medium to a predetermined value, preferably using a closed loop regulation and/or the method comprising a step of controlling a counterpressure at an outlet of the culture system to adjust the flow rate of the liquid medium from the liquid reservoir to the culture system, preferably using a closed loop regulation, or the method comprising controlling a hydraulic resistance downstream of the liquid reservoir to adjust the flow rate of the liquid medium from the liquid reservoir to the culture system, preferably using a closed loop regulation.

In some variations, liquid medium flowed to the culture system has a concentration of dissolved oxygen of approximately 18.6% or less, preferably of approximately 10% or less, more preferably of approximately 5% or less.

In some variations, the liquid medium flowed to the culture system contains one or more components selected from nutrients, drugs such as chemotherapy drugs, immunotherapy drugs, hormonotherapy drugs and inhibitors.

In some variations, the method comprises taking at least one measurement wherein the liquid medium has a concentration of dissolved gas, preferably oxygen, at a first value; and at least one measurement wherein the liquid medium has a concentration of dissolved gas, preferably oxygen, at a second value different from the first value.

In some variations, the method comprises collecting liquid medium from another culture system and feeding it to the at least one liquid reservoir prior to flowing the liquid medium from said liquid reservoir to the culture system.

In some variations, the method comprises an incubation step wherein liquid medium devoid of a test compound is flowed to the culture system, followed by a test step wherein liquid medium comprising a test compound is flowed to the culture system, optionally followed by a rinsing step wherein liquid medium devoid of the test compound is flowed to the culture system.

The invention also relates to an assembly, comprising:

- a culture system;
- a feeding device fluidically connected to the culture system, the feeding device comprising at least one feeding module, and each feeding module comprising:

  - a liquid reservoir containing a liquid medium and provided with a gas inlet, a gas outlet and a liquid outlet,
  - an admission line configured to provide a gaseous medium into the liquid reservoir, the admission line being connected to the gas inlet, and
  - an emission line configured to withdraw the gaseous medium from the liquid reservoir, the emission line being connected to the gas outlet;

  the feeding device further comprising at least one control valve associated with the admission line(s) and at least one control valve associated with the emission line(s);
- at least one sensor for measuring the presence or concentration of at least one substance in the liquid medium, positioned inside the culture system, and/or at least two sensors for measuring the presence or concentration of at least one substance in the liquid medium, placed upstream of the culture system and downstream of the culture system.

In some variations, the at least one culture system is a cell culture system, preferably selected from a microfluidic cell culture system, a millifluidic cell culture system or a nanofluidic cell culture system.

In some variations, the sensor(s) comprise at least one metabolic sensor, preferably selected from glucose sensors, lactate sensors and/or amino acid sensors; and/or the sensor(s) comprise at least one pH sensor and/or oxygen sensor.

The present invention makes it possible to address the need mentioned above. In particular, the invention provides a method for monitoring at least one substance, which may be produced or consumed by cells or other living entities, that is more efficient and more versatile.

This is achieved by providing the culture system with a liquid medium with a controlled concentration of gas (e.g., oxygen) and at a controlled flow rate, so that the *in vitro* conditions can be configured to closely replicate those of the desired *in vivo* conditions. Moreover, the method allows for the determination of the presence or concentration of at least one substance in the liquid medium in or downstream (and possibly upstream) of the culture system. This can include, for example, performing a measurement of cell consumption or secretion. Additionally, the method may be used to directly determine the presence or consumption of a wide range of substances. In other words, the method allows for complete monitoring of a culture system while maintaining control of injected liquid medium.

Advantageously and according to some embodiments, the method may comprise taking a measurement or multiple successive measurements within the culture system. This allows for substances to be monitored within the culture system itself over time. Additionally or alternatively, the method may comprise taking a measurement or multiple successive measurements upstream and downstream of the culture system to directly determine the production or consumption of a substance in the culture system.

Additionally or alternatively, a number of embodiments employ at least two feeding modules and/or multiple culture systems along with control valves associated with all relevant admission and emission lines. This allows for a flexible and diverse range of configurations for which culture systems can be accurately controlled and monitored.

## Brief description of the drawings

FIG. 1 schematically shows an example of an assembly which can be used for implementing the invention.
FIG. 2 schematically shows another example of an assembly which can be used for implementing the invention.
FIG. 3 schematically shows an example of an implementation of the method in the assembly of FIG. 2.
FIG. 4 schematically shows another example of an assembly which can be used for implementing the invention.
FIG. 5 schematically shows another example of an assembly which can be used for implementing the invention.
FIG. 6 schematically shows another example of an assembly which can be used for implementing the invention.
FIG. 7 schematically shows an example of an implementation of the method in the assembly of FIG. 6.
FIG. 8 schematically shows another example of an assembly which can be used for implementing the invention.
FIG. 9 schematically shows an example of an implementation of the method in the assembly of FIG. 8.
FIG. 10 schematically shows another example of an assembly which can be used for implementing the invention.
FIG. 11 schematically shows another example of an assembly which can be used for implementing the invention.
FIG. 12 schematically shows a detail of the assembly of FIG. 11.

## Detailed description

The invention will now be described in more detail without limitation in the following description.

### General principle

The method of the present invention is implemented by monitoring at least one substance which may be produced or consumed by at least one living entity.

A *"living entity'* refers to any entity showing certain attributes including responsiveness, growth, metabolism, energy transformation, aging, stem cell fate, immunology response and/or reproduction. Preferably, the term may refer to any entity capable of the processes of consumption and secretion.

The at least one living entity may in particular be a population of individual cells, including combinations of different type of cells, organoids, cellular assemblies, organelles, organs-on-chips, tissue slices, and unicellular or multicellular organisms. The cells may include, as an exemplary and non-exhaustive list, eukaryote cells, including animal cells (such as mammal cells and more specifically human cells), yeast cells, fungal cells, plant cells, protozoa; prokaryote cells, such as bacteria. Any combination of the above may also be used. Multicellular organisms may include animals, notably but not exclusively, laboratory model animals such as nematodes, embryos, notably non-human embryos (such as fish embryos), flies, eggs, plants, fungi, genetically modified organisms (GMOs). A cell culture may also be performed in order to grow viruses (i.e., the cells may be virus-containing cells). The at least one living entity may be a sample of biological tissue, such as in particular a tumoral tissue. The cells of the at least one living entity may be arranged in 2D (i.e. monolayer) or 3D form.

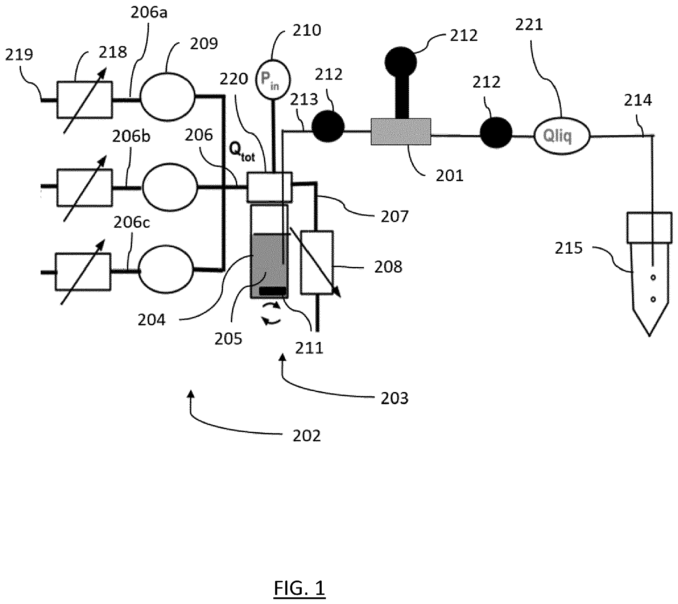

Making reference to FIG. 1, the method of the invention is implemented in an assembly comprising a culture system 201 and a feeding device 202 fluidically connected to it.

In some embodiments, the feeding device 202 and the culture system 201 are integrated as a single component, such as a cartridge. Otherwise, the culture system 201 is a component separate from the feeding device 202, and fluidically connected to it, e.g., by tubing.

The cartridge can be made from a polymer material or from any non-polymer material such as glass known by those skilled in the art to prepare solid components. Typical, non-exhaustive, examples of materials usable for the cartridge are elastomers, thermoplastics, resins, glass, fused silica, or silicone. As for elastomers, they can be for instance, and non limitatively silicones such as polydimethylsiloxane, polyurethanes, acrylic elastomers, fluoroelastomers, polyenes, and the like. Materials marketed under the brand Tygon® may especially be useful. As for thermoplastic polymers, they can be for instance and non limitatively polyolefins, such as polyethylene, polypropylene, and more generally polyenes and their copolymers, low or high density, crosslinked or not, cyclic olefin polymers, cyclic olefin copolymers, acrylates such as polymethylmethacrylates, polycarbonates, polyesters, fluorinated polymers, and the like. Devices of the invention may also be prepared using resins, notably epoxy, polyester, polyurethane resins. The cartridge may also comprise combinations of the above materials. The cartridge may be manufactured by any fabrication method known in the art, such as molding, embossing, additive or subtractive manufacturing, machining and micromachining, photolithography, and the like. Any lines, tubes, or more generally components linking the liquid reservoir and the culture system can be themselves integrated into the single piece or cartridge. In particular, lines connecting the liquid reservoir to the culture system may be molded fluidic lines within the single piece or cartridge.

The culture system 201 contains the at least one living entity.

By *"culture system"* is meant a system in which the living entity is loaded and cultured.

The culture system 201 comprises at least one channel through which the liquid medium provided by the feeding device 202 can flow. According to some embodiments, the culture system 201 may comprise more than one channel. The culture system 201 is preferably gas-tight. Thus, the culture system 201 should preferably be made of a material which is substantially non-permeable to gases. Such material may be chosen from glass, poly(methyl methacrylate) (PMMA), metal, polypropylene, polycarbonate, polystyrene, cyclic olefin polymers or copolymers (COC), polyether ether ketone, perfluorocarbons or fluorocarbons, or more generally thermoplastic materials and polymers. In some embodiments, said member may be an elastic material, such as elastomers, siloxane elastomers, polydimethylsiloxane, natural rubber, polyenes such as notably polyisoprene and its copolymers, polybutadiene, thermoplastic elastomers, fluorinated elastomers, polyurethanes, polyacrylates, epoxies. A preferred material for the culture system is a material with a low gas permeability, and notably glass or fused silica.

The culture system 201 may for example be a cell culture system, preferably selected from a microfluidic cell culture system, a millifluidic cell culture system or a nanofluidic cell culture system.

By *"millifluidic cell culture system"* is meant a cell culture system in which the minimal channel or chamber dimensions are of the order of 1-10 mm.

By *"microfluidic cell culture system"* is meant a cell culture system in which the minimal channel or chamber dimensions are of the order of 1 to less than 1000 $\mu$m.

By *"nanofluidic cell culture system"* is meant a cell culture system in which the minimal channel or chamber dimensions are of the order of less than 1 $\mu$m.

Culture systems may be made by microfabrication, or by the assembly of microcapillaries, or more generally by any method able to provide systems with channels of the specified dimensions. Also, they are not restricted to two-dimensional *"chips",* and encompass any 2-D or 3-D geometries of such channels, chambers, or arrays of channels or chambers or any combination thereof.

According to some embodiments, the culture system 201 may be an organ-on-chip or a tissue-on-chip. By *"organ-on-a-chip"* or by *"tissue-on-a-chip"* is meant a multi-channel 2-D or 3-D microfluidic cell culture chip that simulates the activities, mechanics and physiological response of entire organs, organ systems or tissues.

The culture system 201 is preferably devoid of separate gas flow elements. Thus, the culture system 201 may be devoid of a gas channel, or a gas chamber or a gas-permeable material (such as a membrane). This makes it possible to reduce fabrication cost and simplify the fabrication process.

The culture system 201 may be placed in an oven, so that it remains at a controlled temperature, for instance a constant temperature of approximately 37°C. The oven may be part of the assembly. In some other embodiments, temperatures can be controlled by a temperature control system directly integrated in the culture system 201, comprising for instance a Peltier element, or a thermalization fluid circulation, or a resistor, or a temperature sensor, or any combination thereof.

The feeding device 202 is fluidically connected to the culture system 201. The feeding device 202 comprises at least one feeding module 203. In what is depicted in FIG. 1, a single feeding module 203 is provided.

The feeding module 203 comprises a liquid reservoir 204 containing a liquid medium 205. The liquid medium 205 is preferably an aqueous solution. It may, for example, comprise at least one reagent, and/or at least one reactant, and/or at least one energy source (e.g. glucose, glutamine, acetyl-CoA), and/or a drug or other medical treatment, and/or at least one inhibitor, such as a metabolic inhibitor (e.g. mitochondrial translation inhibitor). Some examples of inhibitors are: 2-deoxy-D-glucose a.k.a. 2-DG as a glucose metabolism inhibitor, etomoxir as a fatty acid metabolism inhibitor, CB-839 as a glutamine metabolism inhibitor, $\alpha$-CHCA or AZD3965 as a lactate metabolism inhibitor, trifluoromethoxy carbonylcyanide phenylhydrazone a.k.a. FCCP, carboxin, cyanide, rotenone, oligomycin and antimycin A as mitochondrial inhibitors. The liquid medium 205 is preferably a growth medium, i.e. an aqueous composition adapted to support the growth of the at least one living entity, e.g., to support the growth of a population of microorganisms or cells via the process of cell proliferation.

The liquid reservoir 204 is provided with a gas inlet, a gas outlet, and a liquid outlet. An admission line 206 is configured to provide a gaseous medium into the liquid reservoir 204, the admission line 206 being connected to the gas inlet of the liquid reservoir 204, and an emission line 207 is configured to withdraw the gaseous medium from the liquid reservoir 204, the emission line 207 being connected to the gas outlet of the liquid reservoir 204.

Optionally, the liquid reservoir 204 may be provided with a closable liquid inlet, in order to refill the liquid reservoir 204 or modify the composition of the liquid medium 205 if desired.

The liquid reservoir 204 may be supplied with a cap 220 to seal it from the environment. The gas and liquid inlets and outlets described herein may comprise respective passageways within the cap 220. A sealing member may be provided between the cap 220 and the reservoir body, such as an O-ring, an elastomer gasket or any deformable part. The sealing member may be squeezed between the cap 220 and the reservoir body.

The feeding device 202 further comprises at least one control valve 218 associated with the admission line 206 and at least one control valve 208 associated with the emission line 207.

The admission line 206 is connected to at least one gas source 219. According to some embodiments, the or each gas source 219 may be a container containing a pressurized gas (or a gas composition). The or each gas source 219 may provide a controlled gas composition, a controllable pressure, a controllable flow rate, or any combination thereof. Preferably, the or each gas source 219 provides a predetermined gas composition at a pressure which may be controlled owing to a regulator on the gas source 219. Alternatively, the gas source 219 may be a gas mixer, or in other words a container comprising at least two pressurized gases (or gas compositions) separated from one another. In this case, a mixture of the separated gases can be provided into the liquid reservoir 204 via the admission line 206. It is preferable that such gas mixer is a Venturi gas mixer, or a tunable gas mixer, so that the composition of the gaseous mixture fed to the admission line 206 can be adjusted by the user.

The gas or gas composition in the gas source(s) 219 may be chosen from air, oxygen, nitrogen, carbon dioxide, carbon monoxide, nitric oxide, hydrogen sulfide or any other gases.

In some embodiments (not shown), a plurality of admission lines 206 may be connected to respective gas inlets on the liquid reservoir, each admission line being also connected to a respective gas source 219, and a respective control valve being associated with each admission line 206. A flow meter 209 may also be provided on each admission line 206. This makes it possible to adjust the composition of the gaseous medium provided to the liquid reservoir 204, by acting on the respective control valves 218 of the various admission lines 206. For instance, two gas sources 219 may independently provide oxygen and carbon dioxide; or oxygen and nitrogen; or nitrogen and carbon dioxide; or air and oxygen; or air and carbon dioxide; or air and nitrogen. In other examples, three gas sources 219 may independently provide oxygen, carbon dioxide and nitrogen; or air, carbon dioxide and nitrogen.

Alternatively (or additionally), as shown on FIG. 1, one admission line 206 may branch out into a plurality of admission branches 206a, 206b, 206c, so as to be connected to multiple gas sources 219, each gas source 219 preferably providing a different gas. All parallel branches 206a, 206b, 206c are joined in order to provide a single (mixed) flow of gaseous medium through the gas inlet of the liquid reservoir 204. A gas tank may be provided between the gas sources 219 and the gas inlet of the liquid reservoir 204, configured to mix the different gases exiting the different gas sources 219 prior to the entrance of the gas mixture into the liquid reservoir 204. For instance, two gas sources 219 may independently provide oxygen and carbon dioxide; or oxygen and nitrogen; or nitrogen and carbon dioxide; or air and oxygen; or air and carbon dioxide; or air and nitrogen. In other examples, three gas sources 219 may independently provide oxygen, carbon dioxide and nitrogen; or air, carbon dioxide and nitrogen. Each admission branch 206a, 206b, 206c may be provided with a respective control valve 218. Each admission branch 206a, 206b, 206c may also comprise a respective gas flow meter 209 to monitor the flow of the gaseous medium in the branch. An additional flow meter (not illustrated) may be provided on the admission line 206 downstream of the junction of the branches 206a, 206b, 206c, in order to monitor (and thus control) the total flow rate of the gaseous medium entering the liquid reservoir 204. Using the individual flow meters 209 and control valves 218 on each branch 206a, 206b, 206c and optionally the additional flow meter 209 downstream of the junction, it is possible to adjust the composition of the gaseous medium fed to the liquid reservoir 204 as well as its flow rate.

Each control valve 218 associated with the admission line(s) 206 may preferably be an electro-valve. In some embodiments, the admission line 206 may also comprise at least one check valve to prevent backflow of the liquid present in the liquid reservoir 204 in the admission line 206.

An electro-valve is capable of achieving and maintaining different openings depending on the applied voltage.

Alternatively, if the control valve 218 is not an electro-valve, it can be a pneumatically actuated valve, or a mechanically actuated valve.

According to some embodiments, the control valve 218 is a continuously controllable valve. By "continuously controllable valve" is meant a valve which can adopt a series of multiple (i.e. more than 2) opening values or a series of multiple values of flow resistance, in response to different control signals. There can be a finite number of opening values or values of flow resistance, or there can be a continuous series of such values.

Optionally, the feeding device 202 may further comprise at least one pressure regulator between the gas source 219 and the control valve 218, configured to regulate the pressure of the gas entering the liquid reservoir 204.

In some embodiments, the temperature of the gas can be controlled by a temperature control system directly integrated in the admission line 206, comprising for instance a Peltier element, or a thermalization fluid circulation, or a resistor, or a temperature sensor, or any combination thereof. The temperature of the gas can also be controlled with an exchanger inside an oven. This allows to equilibrate the liquid source with a gas source 219 at a set temperature (e.g., approximately 37°C), otherwise although the liquid source itself is at the set temperature, the liquid in the system could be at a different temperature.

Furthermore, the admission line 206 may comprise one or more than one sensor chosen from a pressure sensor, a temperature sensor, a concentration sensor, a flow sensor, a light sensor, a camera, or a current or voltage sensor.

The feeding device 202 may preferably comprise a single emission line 207 at the gas outlet. The gaseous medium flows into the at least one liquid reservoir 204 via the admission line(s) 206 and out of the at least one liquid reservoir 204 via the emission line 207, so as to apply a pressure in the at least one liquid reservoir 204. The outlet of the emission line 207 may be at the atmosphere. Alternatively, the emission line 207 may be connected to a pressure controller which makes the system independent of variations of atmospheric pressure. This is useful since the concentration of dissolved gas in a liquid medium depends on absolute pressure, not on relative pressure.

By "pressure controller" is meant any device (including, e.g., valves, pressure sensors and regulators, gas containers and any hydrostatic pressure source) that maintains a pressure at a target absolute value (using e.g. an absolute pressure sensor rather than a gauge sensor referenced at the atmospheric pressure).

The emission line 207 comprises at least one control valve 208 (also designated below as an emission valve). Such control valve 208 may be located between the gas outlet of the liquid reservoir 204 and the pressure controller (if present). Alternatively, the control valve 208 may be integrated within the pressure controller itself. The control valve 208 may preferably be an electro-valve, as described above. Alternatively, if the control valve 208 is not an electro-valve, it can be a pneumatically actuated valve, or a mechanically actuated valve.

According to some embodiments, the control valve 208 is a continuously controllable valve (as defined above). The liquid reservoir 204 may preferably comprise at least one inlet configured to receive a sensor. Such sensor may be a pressure sensor 210 (as illustrated on FIG. 1), a temperature sensor, a concentration sensor, a pH sensor, a flow sensor, a light sensor, a camera, or a current or voltage sensor. This makes it possible to directly measure a specific property (such as temperature or pressure for example) in the liquid reservoir 204.

According to some embodiments, the liquid reservoir 204 comprises a single inlet configured to receive a sensor. In this case, it is preferable that the sensor is a pressure sensor 210.

According to other embodiments, the liquid reservoir 204 comprises more than one inlet configured to receive a sensor. In this case, at least one of these inlets is preferably configured to receive a pressure sensor 210.

Therefore, the feeding device 202 may further comprise a sensor chosen from a pressure sensor, a temperature sensor, a concentration sensor, a flow sensor, a pH sensor, a light sensor, a camera, or a current or voltage sensor, and preferably a pressure sensor, extending into the liquid reservoir 204.

In some embodiments, a pressure sensor may be present in a location other than the liquid reservoir 204, for example between the admission valve 218 and the emission valve 208. The pressure sensor may for example be arranged on the admission line 206.

Pressure in the liquid reservoir 204 may be monitored by a pressure sensor 210 as described above.

Furthermore, the emission line 207 may comprise one or more than one sensor chosen from a pressure sensor, a temperature sensor, a concentration sensor, a flow sensor, a light sensor, a camera, or a current or voltage sensor.

For example, the emission line 207 may comprise at least one flow meter (not illustrated) in order to measure the flow rate of the gaseous medium withdrawn from liquid reservoir 204. This flow meter may be located between the gas outlet of the liquid reservoir 204 and the pressure controller. This may be in addition to or instead of the flow meter(s) on the admission line 206.

The or each gas inlet may comprise a tube (or capillary) extending into the liquid reservoir 204. In other words, the gas inlet may comprise a tube having two open extremities, a first open extremity which can be connected to the admission line 206 and a second open extremity located inside the liquid reservoir 204. It is preferable that the second open extremity is located in a lower (bottom) part of the liquid reservoir 204 in order to provide (and thus bubble) the gaseous medium in the liquid medium 205 present in the liquid reservoir 204, even when only a small amount of liquid medium 205 is found into the liquid reservoir 204. In particular, it is preferable that said second extremity is located in the liquid reservoir 204 below the location of the entry point of the gas outlet in said liquid reservoir 204. In other words, the tube of the gas inlet is preferably immersed in the liquid medium 205. This makes it possible to bubble the gas in the liquid medium 205 present in the liquid reservoir 204. However, in this case, the feeding device 202 may preferably comprise one or more valves so as to avoid any flow of liquid (backflow) out of the liquid reservoir 204 through the gas inlet and through the admission line 206.

In some embodiments, the second open extremity may be provided with a nozzle. The presence of the nozzle makes it possible to reduce the size of bubbles gassed into the liquid medium inside the liquid reservoir 204 and thus increase the surface of diffusion between the gas and the liquid medium 205.

In some embodiments, the gas inlet may enter the liquid reservoir 204 directly at its lower (bottom) part.

The gas outlet may comprise a tube (or capillary) extending into the liquid reservoir 204. In other words, the gas outlet may comprise a tube having two open extremities, a first open extremity which can be connected to the emission line 207 and a second open extremity located in the liquid reservoir 204. It is preferable that the second open extremity is located in an upper (top) part of the liquid reservoir 204 in order to withdraw the gaseous medium from the liquid reservoir 204, even when the liquid reservoir 204 is substantially full of liquid medium 205. The tube of the gas outlet is not immersed in the liquid medium 205.

The liquid outlet may comprise a tube (or capillary) extending into the liquid reservoir 204. In other words, the liquid outlet may comprise a tube having two open extremities, a first open extremity which can be connected to the liquid inlet of the culture system 201 (for example via a fluidic line) and a second open extremity located inside the liquid reservoir 204, so that the tube is immersed in the liquid medium 205. It is preferable that the second open extremity is located at a lower (bottom) part of the liquid reservoir 204 in order to withdraw liquid from the liquid reservoir 204, even when only a small amount of liquid medium 205 is found into the liquid reservoir 204.

In some embodiments, and as shown on FIG. 1, the liquid reservoir 204 may comprise an agitating element 211 such as a magnetic stirrer, and mechanical stirrer, an ultrasound or sound transducer, a vibrating element. This makes it possible to mix the gas entering the liquid reservoir 204 with the liquid present in the liquid reservoir 204 without necessarily bubbling the gas in the liquid.

In the method of the invention, the liquid medium 205 flows with a controlled concentration of a dissolved gas and at a controlled flow rate, from the at least one liquid reservoir 204 to the culture system 201 due to the pressure in the at least one liquid reservoir 204.

A *"concentration of a dissolved gas"* refers to the quantity of gas dissolved in a given volume of liquid at a constant temperature. According to Henry's law the quantity of gas which dissolves in a given volume of liquid is directly proportional to its partial pressure P in equilibrium with the liquid.

The concentration of dissolved gas(es) in the liquid medium 205 depends on the composition of the gaseous medium and on the pressure in the liquid reservoir 204, thus adjusting the pressure or the composition of the gaseous medium allows to control and modify (if necessary) the concentration of one or more dissolved gases in the liquid medium 205. The pressure, in turn, can be adjusted by controlling the valves 218, 208 of the admission and emission lines 206, 207. The composition of the gaseous medium can also be modified by independently controlling the valves 218 of the admission line(s) 206 (if there are two or more independent gas sources 219) or by controlling the gas source 219 itself.

The gas flow rate may for example be from 2 mL/min to 5 L/min. According to some preferred embodiments, the gas flow rate may be from 50 mL/min to 700 mL/min. According to other preferred embodiments, the gas flow rate may be from 2 mL/min to 50 mL/min. According to other preferred embodiments, the gas flow rate may be from 500 mL/min to 5 L/min.

By controlling the control valve(s) 218 on the admission line(s) 206 and the control valve 208 on the emission line 207, it is possible to adjust the flow rate of gaseous medium into and out of the liquid reservoir 204, and the pressure in the liquid reservoir 204.

In turn, the pressure inside the liquid reservoir 204 determines the concentration of dissolved gas(es) in the liquid medium 205 contained in the liquid reservoir 204.

The concentration in a dissolved gas is calculated based on the following equation:

$$[\text{dissolved gas component}] = K_h \times \text{proportion of gas component in gaseous medium} \times P$$

$K_h$ being Henry's constant and P being the total pressure in the liquid reservoir 204.

The flow rate of the liquid medium 205 can be adjusted depending on the pressure in the liquid reservoir 204 and the counter-pressure at the liquid outlet of the culture system 201. The liquid medium 205 flow rate may be for example from 0.5 µL/min to 200 µL/min (in particular for organ-on-chip applications) or 200 µL/min to 50 mL/min or 50 to 100 mL/min.

The liquid medium 205 may be provided from the liquid reservoir 204 to the culture system 201 via a feeding line 213 connected to the liquid outlet of the liquid reservoir 204 and to a liquid inlet of the culture system 201. A valve may be present at the liquid outlet of the liquid reservoir 204 or on the feeding line 213 in order to open or close the fluidic connection between the liquid reservoir 204 and the culture system 201 (so as to be able to stop the flow of liquid medium). At an outlet of the culture system 201, liquid medium may be collected via at least one collecting line 214. The collecting line 214 may lead to at least one collecting reservoir 215.

Each of the feeding line 213 and collecting line 214 may comprise tubing, or may comprise a (e.g. molded or microfabricated) channel if it is integrated together with the reservoir 204 and/or the culture system 201 within a module or cartridge.

A liquid flow sensor or flow meter 221 may be provided on the or each feeding line 213.

A liquid flow sensor or flow meter 221 may be provided on the or each collecting line 214.

According to some embodiments, the culture system 201 comprises a single liquid inlet. In this case, the liquid outlet of the liquid reservoir 204 is connected to the liquid inlet of the culture system 201 via the feeding line 213 in order to provide the at least one channel of the culture system 201 with liquid medium having a controlled flow rate and a controlled concentration in a dissolved gas.

According to other embodiments, the culture system 201 comprises two or more liquid inlets. In this case, the liquid outlet of the liquid reservoir 204 may be connected to all liquid inlets of the culture system 201 (for example in order to provide the two or more channels of the culture system 201 with the liquid medium 205 having a controlled flow rate and a controlled concentration in a dissolved gas), or each liquid inlet of the culture system 201 may be connected to a different liquid outlet of the same liquid reservoir 204 (still in order to provide each channel of the culture system 201 with the liquid medium 205 having a controlled flow rate and a controlled concentration in a dissolved gas), via one or more feeding lines 213.

The liquid outlet of the culture system 201 may be at ambient (atmospheric) pressure. Alternatively, the pressure at the liquid outlet of the culture system 201 may be controlled owing to an outlet gas pressure source which may be the same as the outlet pressure source of the feeding device 202 or which may be different. The set pressure may be below or above atmospheric pressure.

The difference between the pressure in the liquid reservoir 204 and the counterpressure at the outlet of the culture system 201 may for example range from 10 mbar to 7800 mbar, and preferably from 50 mbar to 1000 mbar.

In particular, the collecting reservoir 215 may be connected to a pressure controller via a gas connection. The liquid outlet of the culture system 201 may be connected to a liquid inlet of the collecting reservoir 215 via the collecting line 214. Thus, the liquid medium may exit the culture system 201 and enter the collecting reservoir 215. The pressure controller makes it possible to impose a certain pressure within the collecting reservoir 215. For example, a constant pressure may be maintained in the liquid reservoir 204 and the pressure in the collecting reservoir 215 may be regulated so as to adjust the liquid flow rate.

Alternatively, the collecting reservoir 215 may comprise at least one liquid inlet, at least one gas inlet and at least one gas outlet. In this case, the configuration of the collecting reservoir 215 may be similar to the that of the liquid reservoir 204 described above (with the difference that the collecting reservoir 215 comprises at least one liquid inlet while the liquid reservoir 204 comprises at least one liquid outlet). The liquid outlet of the culture system 201 may be connected to the liquid inlet of the collecting reservoir 215 via the collecting line 214. Thus, the liquid medium 205 may exit the culture system 201 and enter the collecting reservoir 215.

The gas inlet of the collecting reservoir 215 may be connected to the admission line 206.

The gas outlet of the collecting reservoir 215 may be connected to a pressure controller which makes it possible to impose a certain pressure at the gas outlet.

This configuration is especially useful when the assembly comprises a recirculation capability, as will be further described below.

Alternatively to the pressure controller or additionally, the assembly may comprise a flow restrictor (not illustrated). According to some embodiments, more than one flow restrictors can be comprised in the assembly. A flow restrictor may be placed upstream of, within, or downstream of the culture system 201. According to preferred embodiments, the liquid inlet of the culture system 201 may be connected to a flow restrictor. The flow restrictor (which can be for example, and not limited to, a pinch valve, or a solenoid valve, or a piezo valve, or a passive capillary, or a mechanical valve, or an electro-valve or a filter, preferably a pinch valve) can be located between the liquid outlet of the liquid reservoir 204 and the liquid inlet of the culture system 201, and particularly between the liquid flow sensor (if such sensor is present) and the liquid inlet of the culture system 201. The flow restrictor allows to modify the hydraulic resistance, therefore modifying the liquid flow rate without modifying the concentration of dissolved gas(es) in the liquid medium 205.

In some variations, the method comprises a step of flowing the gaseous medium so as to reach a target dissolved gas concentration in the liquid medium 205, without any flow of liquid medium 205, and then a second step of flowing the liquid medium 205 having the target dissolved gas concentration to the culture system 201. The transition from the first step to the second step may be achieved by opening a valve between the liquid reservoir 204 and the culture system 201. Advantageously, the gas keeps flowing during the second step to ensure that the dissolved gas concentration in the liquid medium 205 remains at the targeted level. In some variations, the gas flow rate in the first step is higher than the gas flow rate in the second step. Therefore, the target dissolved gas concentration may be relatively quickly achieved in the first step, and then the gas flow rate may be lowered to a level which is sufficient to maintain the target dissolved gas concentration and to reduce the gas consumption. By way of example, the gas flow rate in the first step may be from 150 mL/min to 700 mL/min, and the gas flow rate in the second step may be from 1 mL/min to 300 mL/min. In some embodiments, the method may comprise keeping the dissolved gas concentration in the liquid medium 205 substantially constant while varying the flow rate of liquid medium 205. This can be performed by maintaining the pressure inside the liquid reservoir 204 at a substantially constant level, and by varying the counterpressure applied at a liquid outlet.

According to the method of the invention, at least one measurement may be taken so as to determine the presence or concentration of the at least one substance in the liquid medium 205 in or downstream of the culture system.

Such substances may for example be any molecule considered as a nutrient and/or a metabolite. Such substances may for example include sugars (e.g. glucose, galactose, fructose), organic acids (e.g. lactate, pyruvate), salts (e.g. acetate, malate), cytokines, chemokines, amino acids (e.g. glutamine, serine, alanine, aspartate, asparagine, glycine, and/or methionine), fatty acids, phospholipids, cholesterol, acetyl-CoA. The substance may also be a virus. The substance may also be a protein or nucleic acid or a macromolecular assembly, such as extracellular vesicles, exosomes, organites, such as mitochondria.

In some embodiments, the substance is not a gas, and in particular is not a dissolved gas in the liquid medium.

In other embodiments, the substance is a dissolved gas, such as $O_2$ or $CO_2$, and most preferably $O_2$.

The measurement may be taken within the culture system. The measurement may, for example, comprise obtaining a reading from one or more sensors 212. In particular, at least one sensor 212 may be located within the culture system 201 (e.g. the sensor may take measurements directly within at least one channel or chamber in the culture system 201), upstream of the culture system 201 (e.g. along a or the feeding line 213 of the culture system 201 and/or at an inlet of the culture system 201) and/or downstream of the culture system 201 (e.g. along a or the collecting line 214 of the culture system 201 and/or at an outlet of the culture system 201; or in a or the collecting reservoir 215, or by taking one or more samples from a or the collecting reservoir 215 and by analyzing them separately).

In the illustration of FIG. 1, three sensors 212 are provided, one integrated within the culture system 201, one upstream of the culture system 201 on the feeding line 213 and one downstream of the culture system 201 on the collecting line 214. Alternatively, the upstream sensor and/or the downstream sensor may be provided at an inlet, respectively at an outlet of the culture system 201.

The sensors may include optical, optomechanical, electrochemical, electroacoustic, electromechanical or thermoelectric sensors. The sensors may comprise a substrate allowing to capture target molecules. The sensors may include supramolecular sensors.

By *"supramolecular sensor"* is meant a sensor including a molecule having a binding site that is specific for an analyte, wherein the molecule and the analyte form a supramolecular assembly when the analyte binds to the molecule. Analyte binding may be registered through a physical, chemical or biochemical mechanism, such as a colorimetric, fluorescent or electrochemical signal. The binding of the analyte at the binding site may preferably be achieved non-covalently, such as by electrostatic interaction, ionic interaction, cation-Pi interaction, hydrogen bonding, Pi-stacking and van der Waals forces.

The sensors may comprise cameras or other imaging devices, which may in particular be configured to detect fluorescent markers, such as fluorescent antibodies which may be encapsulated in an external matrix (e.g., *via* ELISA testing). In particular, such fluorescent markers may be detected directly in the culture system 201. In particular, fluorescent markers within cells in the culture system 201 may be detected. A sensor may comprise a membrane with fluorescent antibodies. Additionally or alternatively, sensors may include chemosensor arrays, organic field-effect transistors, an analysis chamber and/or ELISA microarrays.

In some embodiments, the method comprises an incubation step, wherein incubation is carried out within the culture system 201. Then, in a subsequent measuring step, liquid medium flows through the culture system 201 at a flow rate which is higher than the flow rate of the liquid medium in the incubation step (optionally, the flow rate in the incubation step may be zero). In particular, the substance to be monitored may be moved from the culture system 201 to a downstream sensor 212 (as described above) owing to the flow rate in the measuring step, thus making it possible to make the measurement. The flow rate in the measuring step may also make it possible to refresh the liquid medium within the culture system 201. Such incubation steps and measuring steps may alternate, e.g. in a cyclic manner.

The measurement may be a direct measurement, such as, for example, that of a nutrient or metabolite concentration (e.g. lactose, glucose and/or an amino acid). By *"direct measurement'* is meant that the sensor (or control unit connected to the sensor) directly outputs a nutrient or metabolite concentration value.

Alternatively, the measurement may be indirect. For instance, by measuring one or more parameters such as pH, oxygen concentration or the like, the concentration of a substance (such as a nutrient or metabolite) may be determined.

In particular, taking measurements of the concentration of one substance (for instance, oxygen) makes it possible to determine the concentration of another substance (such as a nutrient or metabolite) which is to be monitored.

The evolution of the presence or concentration of the at least one substance over time may be determined by taking multiple successive measurements (for example directly in the culture system). Measurements may, for example, be taken in real-time. By comparing measurements taken by the same sensor at different points in time, a temporal evolution in the presence / concentration of the monitored substance may be determined.

By comparing measurements taken by sensors at two different positions, a spatial difference in the presence / concentration of the monitored substance may be determined. By way of example, a measurement may be taken upstream of the culture system 201. This enables an initial state of the at least one substance to be determined. A second measurement may then be taken downstream of the culture system 201. The difference between these measurements may therefore enable the determination of a concentration or amount of the substance that was consumed or produced by the at least one living entity. Additionally or alternatively, the difference may enable detection of a particular activity that may have occurred inside the culture system 201.

Additionally or alternatively, the method according to the invention makes it possible to take measurements while controlling and adjusting the liquid flow rate of the liquid medium 205 in the culture system 201 (independently from the dissolved gas concentration in the liquid medium 205).

Adjusting the liquid flow rate may have an impact on the shear rate to which the living entity is subjected. Adjusting the liquid flow rate may also have an impact on the extracellular concentration of secreted substances in the culture system. By way of example, by increasing the liquid flow rate, it is possible to determine the secretion rate of a substance as a function of extracellular concentration of this substance and/or of other substances.

Alternatively, the method may comprise taking measurements while varying the concentration of the dissolved gas in the liquid medium 205, either while keeping the liquid flow rate substantially constant, or while varying the liquid flow rate. This can be performed notably by modifying the pressure in the liquid reservoir 204. By simultaneously adjusting if necessary the counter-pressure and/or the hydraulic resistance, the liquid flow rate may be kept substantially constant despite the modification of the pressure in the liquid reservoir 204, or may be adjusted as needed. In some preferred embodiments, the concentration of dissolved oxygen in the liquid medium 205 is varied as described above. This makes it possible to take measurements of substances cultured under different conditions representative of in vivo conditions in various healthy or diseased (e.g. tumoral) living entities.

In some embodiments, measurements may be taken while the concentration of dissolved gas is varied in an intermittent or cyclic manner. This makes it possible, for example, to culture cells under conditions representative of intermittent hypoxia as can be found in sleep apnea and myocardial infarction. When more than one gas sources 219 are used, the concentration of the dissolved gases in the liquid medium 205 may also be modified (instead of, or in addition to modifying the pressure in the liquid reservoir 204), by modifying the composition of the gaseous medium flowing to the liquid reservoir 204. This can be performed by individually controlling the control valves 218 associated with the various gas sources 219; or by switching between different gas sources 219 comprising different gas compositions. In some embodiments, the gaseous medium comprises oxygen and carbon dioxide, the proportion of which may be independently controlled as described above (using at least two or three separate gas sources 219). This makes it possible to independently control the concentration of dissolved oxygen and dissolved carbon dioxide in the liquid medium 205. The control of the concentration of dissolved carbon dioxide, in turn, makes it possible to control and adjust the pH of the liquid medium 205 (for example at or close to the normal physiological value of 7.4, or at or close to a different value, such as less than 7.4, in order to simulate a disease condition).

The method of the invention may comprise flowing a liquid medium to the culture system 201 wherein the oxygen concentration in the liquid medium is from 0 to 21%, preferably from 0.1 to 15%, or from 0.2 to 10%, or from 0.5 to 5%, or from 1 to 3%. The oxygen concentration may be approximately 18.6%, for example to simulate hyperoxic conditions. The oxygen concentration may be less than 18.6%, preferably less than 5%, for example to simulate physioxic or hypoxic conditions.

The method may comprise flowing a liquid medium to the culture system 201 wherein the $CO_2$ concentration in the liquid medium is from 0 to 20%, preferably from 0.1 to 15%, or from 5 to 10%. Such $CO_2$ concentrations may be selected to obtain a certain pH value of the liquid medium, such as for example approximately 7.4 to simulate physiological conditions, or less than 7.4, or less than 7.2, such as approximately 6.9 to simulate oncological conditions.

A dissolved gas (e.g., oxygen) concentration in the liquid medium, corresponds to the partial pressure of dissolved oxygen in the liquid medium, divided by the total pressure of all dissolved gases in the liquid medium.

Control of the assembly

The assembly may be connected to or may comprise a control unit. The control unit makes it possible for a user to control one or more than one parameter, such gas flow rate, liquid flow rate, dissolved gas concentration, pressure, etc. The control unit may comprise a graphical user interface (GUI) which allows to choose an input value for one or more than one parameter. The control unit may ensure a fully automated operation of the assembly.

The control unit may comprise one or more processors coupled to a storage medium, as well as a computer program comprising instructions stored thereon, for performing various steps described in more detail below. The control unit may receive input from any combination of the sensors mentioned above, as well as input from the user. In particular, the control unit may receive input from one or more of: a pressure sensor associated with a liquid reservoir, at least one gas flow sensor on an admission line, at least one gas flow sensor on an emission line, a liquid flow sensor and optionally a dissolved gas sensor for the liquid medium. The control unit may also receive input from any other pressure sensor, flow sensor, light sensor, pH sensor, camera, current or voltage sensor which may be present in the assembly.

The control unit may process the input data and/or the user instructions and as a result, provide instructions to the various control valves and pressure controllers, and in particular to the control valve(s) 218 on the admission line(s) 206, the control valve(s) 208 on the emission line 207 and the pressure controller connected to the emission line 207, if present. It may also provide instructions to the pressure controller connected to the collecting reservoir 215 (if present) and/or to the flow restrictor (if present).

The control unit may in particular control the aperture ratios of the various control valves.

The aperture ratio k of each control valve 218, 208 can be defined as a ratio of the gas flow rate Q through the valve to the maximum gas flow rate $Q_{max}$ which can be achieved. As a result, when the valve is fully closed, k=0 and when it is fully open, k=1.

If there is one admission valve 218 and it is closed ($k_a$=0) or if the emission valve 208 is closed ($k_e$=0), the gas flow rate is zero. If both of the valves 218, 208 are completely open (ka=ke=1), the flow rate is maximal (Q=Qmax). Changing the opening ratio of the two valves 218, 208 allows to obtain different flow rates. The theoretical approximative relationship between the admission ratio ($k_a$), the emission ratio ($k_e$) and the gas flow rate (Q), at steady state and assuming the gas flow is significantly higher than the liquid flowing out of the liquid reservoir 204, is the following:

$$k_e = \frac{k_a * Q}{k_a * Qmax - Q}$$

Therefore, controlling the admission valve 218 and the emission valve 208 makes it possible to adjust the flow rate of gaseous medium through the liquid reservoir 204.

Similar considerations apply when two or more admission lines 206 are present, with respective admission valves 218.

The aperture ratios also make it possible to control the pressure P in the liquid reservoir 204. If the admission valve 218 is closed and the emission valve 208 is open, P is equal to the outlet pressure of the emission valve 208 $Pout_{gas}$ (atmospheric pressure or pressure set by a pressure controller). If the admission valve 218 is open and the emission valve 208 is closed, P is equal to $P_{max}$, namely the input gas pressure set by the gas source 219. If both valves are open, the theoretical relationship is the following:

$$k_e = k_a * (\frac{Pmax - Pout_{gas}}{P - Pout_{gas}} - 1)$$

In this equation, $P_{max}$ is the input gas pressure (set by the gas source 219), $Pout_{gas}$ is the outlet pressure of the emission valve 208 (atmospheric pressure or pressure set by the pressure controller). In some embodiments, $P_{max}$ may be set by the user or controlled by the control unit by adjusting a regulator or a pump on the or each gas source 219.

As a result, by controlling both the admission valve 218 and the emission valve 208, it is possible to adjust the flow rate of gas Q through the liquid reservoir 204, as well as the pressure P inside the liquid reservoir 204.

As a reminder, the concentration of dissolved gas in the liquid medium 205 varies depending on the pressure P in the liquid reservoir 204.

Besides, the flow rate of liquid medium 205 exiting the liquid reservoir 204 depends on the difference between the pressure P in the liquid reservoir 204 and the pressure $Pout_{liq}$ at the outlet of the culture system 201 (counter-pressure), which in some embodiments can be adjusted by the associated pressure controller.

In summary, by controlling the admission valve 218, the emission valve 208 and pressure controller downstream of the culture system 201, it is possible to independently control the concentration of gas dissolved in the liquid medium 205 (which depends on P) and the liquid flow rate to and through the culture system 201 (which depends on $P - Pout_{liq}$).

By way of example, by maintaining the pressure P in the liquid reservoir 204 constant, and by modifying the counter-pressure at the liquid outlet of the culture system 201, the flow rate of the liquid medium 205 can be varied without modifying the dissolved gas concentration in the liquid medium 205.

In some embodiments, the pressure controller downstream of the culture system 201 can be set to a pressure which may be higher or lower than atmospheric pressure. Using a pressure lower than atmospheric pressure is useful for achieving higher liquid flow rates. Using a pressure higher than atmospheric pressure may be useful to avoid the formation of bubbles in the culture system 201.

In other embodiments, the flow restrictor (such as pinch valve) described above is used to adjust and modify the hydraulic resistance R of the system. Therefore, the flow rate of liquid medium 205 $Q_{liq}$ for a given pressure P in the liquid reservoir 204 may be adjusted even without modifying the counter-pressure $Pout_{liq}$, based on the theoretical relationship $(P-Pout_{liq}) = Q_{liq} \times R$. This may make it possible to simply connect the liquid outlet of the culture system 201 to atmospheric pressure, instead of a pressure controller, and still achieve a control over the flow rate of the liquid medium 205.

Experimentally, deviations are typically observed relative to theoretical relationships described above.

Therefore, advantageously, a regulation algorithm is implemented in the control unit so as to adjust the control of the valves 218, 208, pressure controllers and/or flow restrictor as a function of input from the various sensors (in particular the pressure sensor, liquid flow rate sensor and gas sensor). In some preferred embodiments, said regulation algorithm involves a closed loop configuration. The regulation algorithm may be of the proportional type (P), integral type (I), derivative type (D), proportional-integral type (PI), proportional-derivative type (PD), integral-derivative type (ID) or preferably proportional-integral-derivative type (PID).

In some embodiments, the regulation algorithm may rely on fuzzy logic, artificial intelligence, deep learning or neural networks, to optimize the regulation.

In some embodiments, one or more target values are input by the user or otherwise predetermined, such as a gas flow rate and/or a liquid flow rate and/or one or more concentration of dissolved gas in the liquid medium 205, and the control unit, using the regulation algorithm, and based on inputs from one or more sensors as described above, sends instructions to the control valves, pressure controllers and/or flow restrictor in order to reach the target values as quickly as possible (or within a predetermined timeframe). As an initial phase of the regulation, the control valves 218, 208, pressure controllers and/or flow restrictor may be controlled based on preset values which may be derived from the theoretical relationships described above, or from an experimental calibration of the device, before starting the closed loop regulation per se.

By way of example, the regulation algorithm may include the following steps, in order to adjust the gas flow rate and the pressure in the liquid reservoir 204 to target values (which may be set by the user):

- Open the admission valve 218 and emission valve 208 according to a predefined table, depending on the gas flow rate setpoint.
- Read the sensed gas flow rate.
- Launch a closed loop (e.g. PID) regulation on the admission valve 218 opening in order to reach and maintain the flow rate at or near the desired setpoint.
- Read the sensed pressure.
- Launch a closed loop (e.g. PID) regulation on the emission valve 208 opening in order to reach and maintain the pressure at or near the desired setpoint.

Hence, the gaseous flow rate and the pressure are regulated independently, but consecutively in the same program loop.

As another example, when more than one gas sources 219 are present, the regulation algorithm may include the following steps, in order to adjust the individual gas flow rates and the pressure in the liquid reservoir 204 to target values (which may be set by the user):

- Open the admission valve 218 and emission valve 208 according to a predefined table, depending on the setpoints.
- Read each sensed gas flow rate and launch a closed loop (e.g. PID) regulation on the corresponding admission valve 218 opening in order to reach and maintain the flow rate at or near the desired setpoint.
- Read the sensed pressure.

- Launch a closed loop (e.g. PID) regulation on the emission valve 208 opening in order to reach and maintain the pressure at or near the desired setpoint.

As a variation, the user may for instance input the total gas flow rate as well as the composition of the gas flow, and the control unit determines the setpoint individual gas flow rates required to reach these targets. For example, if three gas containers containing oxygen (or air), nitrogen and carbon dioxide are used, the user may select a target total gas flow rate, and two target gas concentrations in the liquid medium 205 (for example oxygen and nitrogen, or nitrogen and carbon dioxide, or oxygen and carbon dioxide). The control unit then determines the individual gas flow rates required to reach these three target values, and then implements the above regulation algorithm.

In some preferred embodiments, the control unit may use information gained from the analysis of images taken by a camera, e.g., by time-lapse acquisitions. In particular, said images may provide from imaging of the culture system 201. Said analysis may for example provide information about the number, the shape, the metabolism, the dynamics or other properties of the cells in the culture system 201. The control unit may for example allow for live imaging of cell death, cell division, cell dynamics, cell-cell interactions, mitochondrial networks, Reactive Oxygen Species (ROS) levels, and/or transporters/receptors such as GLUT1 for glucose, the SLC38 family for glutamine, and/or CD36 for the fatty acids. The imaging may allow to determine the presence (including at a given location within the culture system, such as within cells themselves) or concentration of any protein, receptor, cytokine, chemokine, organite or vesicle.

The control unit may take information obtained from measurement data and process it to provide a certain status on the GUI. The GUI may for example, display a notification if a certain substance or combination of substances has been detected. It may equally display if a certain concentration of substances or combination of substances has been detected. It may also display concentrations measured at multiple locations, or one or more concentration differences between locations. It may also display concentrations measured at multiple points in time, or one or more concentration differences between various points in time. Depending on the substance or concentration of substance(s) measured, the control unit may implement an algorithm to translate such a measurement into a particular result other than substance or substance concentration detection. Further, the control unit may for example process the measured data to provide instructions to the various control valves and pressure controllers or to adjust the sample sizes measured by the sensors per unit of time.

The control unit may be configured to adjust a dissolved gas (e.g., oxygen) concentration in the liquid medium and/or to adjust the flow rate of the liquid medium depending on the measurements relating to the at least one substance. For example, the dissolved gas (e.g., oxygen) concentration in the liquid medium and/or the flow rate of the liquid medium may be adjusted based on the determined level of consumption of oxygen by the living entity, or based on the determined level of consumption of a nutrient (such as glucose) by the living entity, or based on the determined level of secretion of a metabolite. The adjustment may be adapted so as to maintain said level of consumption or secretion above a predetermined threshold, or below a predetermined threshold, or within a predetermined range. The control unit may be further adapted based on the above principles to implement more complex embodiments including multiplexing or a recirculation capability.

Multiplexing

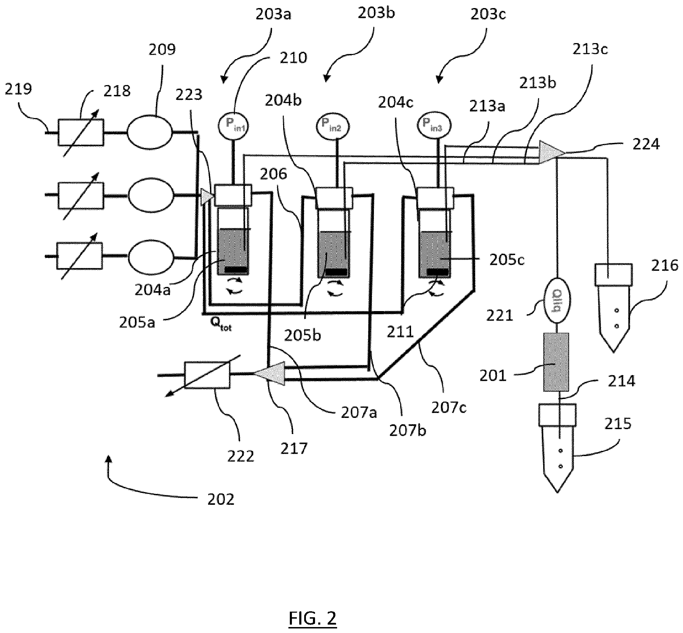

According to some embodiments, and as shown in FIG. 2, the feeding device 202 may comprise at least two feeding modules 203. In the illustrated embodiment, three feeding modules 203a, 203b, 203c are present. Unless explicitly specified, the assembly and in particular each feeding module 203a, 203b, 203c may comprise all features described above in connection with the general principle of the invention.

The feeding modules 203a, 203b, 203c may comprise different liquid media 205a, 205b, 205c in their respective liquid reservoirs 204a, 204b, 204c. Each liquid reservoir 204a, 204b, 204c may be fluidically connected to the culture system 201.

For example, a sequence of inhibitors can be provided to the culture system 201, such as oligomycin, 2-DG, FCCP, rotenone and antimycin A; or 2-DG, etomoxir, CB-839 and/or $\alpha$-CHCA and/or AZD3965. Specific pharmacological treatments can be injected such as chemotherapy, immunotherapy, hormonotherapy etc. treatments, also in combination, and with different injection timing and temporal sequences.

These injections of inhibitors and/or pharmacological treatments can be combined with the use of nutrient-rich or on the contrary nutrient-poor liquid media.

A gaseous medium or different gaseous media may flow directly to each liquid reservoir 204a, 204b, 204c through separate admission lines 206 dedicated to each liquid reservoir 204a, 204b, 204c specifically, as described above in relation to FIG. 1. As shown in FIG. 2, one or more gas sources 219, each being equipped with a flow meter 209 and admission valve 218 may be connected to the admission lines 206 of all feeding modules 203a, 203b, 203c. An admission switching module 223 makes it possible to orient the gaseous medium for the gas source(s) 219 to the admission line 206 of a feeding module. In some embodiments, the gaseous medium may flow in the admission line 206 of only one feeding module 203 at a time. In other embodiments, the gaseous medium may simultaneously flow in the admission lines 206 of more than one feeding module 203.

Generally, by *"switching module"* is meant a module comprising a number of inlet and outlet ports, which has a number of different configurations allowing to selectively direct fluid from at least one inlet to at least one inlet. A switching module may comprise one inlet port and several outlet ports, or several inlet ports and one outlet port, or several inlet ports and several outlet parts. A switching module may be composed of a single valve (e.g., a rotating valve comprising a stator and a rotor, with channels provided in the stator and/or in the rotor), or may comprise several valves which may be interconnected (e.g. via tubes, or mounted on a manifold). A switching module may comprise one or more check valves to prevent any backflow.

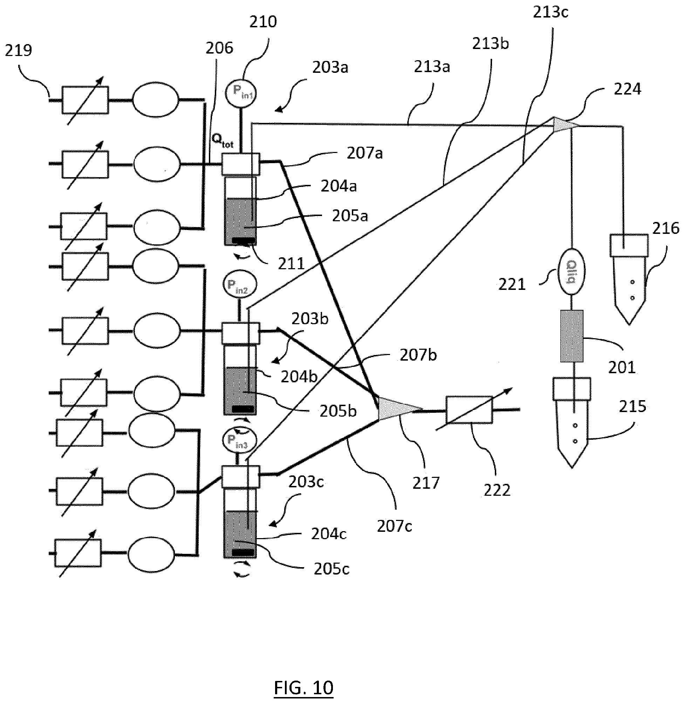

The admission switching module 223 may be a selection valve allowing to fluidically connect one inlet port to one or more outlet ports. Alternatively, and as shown in FIG. 10, each admission line 206 may be connected to different (dedicated) gas source(s) 219. In this case, each feeding module 203a, 203b, 203c can operate under an even more flexible range of different flow rates, pressures and gas concentrations. This in turn allows for the living entity in the culture system 201 to be tested under more complex conditions that may even more closely represent the desired in vivo environment (e.g. injection of a sequence of metabolic inhibitors, a sequence of treatments and/or a sequence of energy sources such as glucose, glutamine and/or acetyl-CoA). Preferably, such a sequential method may be used to combine various inhibitors with chemotherapeutic drugs. The presence or concentration of a substance such as, for example, lactate or glucose, may be detected or measured in and/or downstream of the culture system 201.

In both cases (common gas sources 219 or dedicated gas sources 219 for each feeding module), the gaseous medium may exit each liquid reservoir 204a, 204b, 204c via respective emission lines 207a, 207b, 207c. Each emission line 207a, 207b, 207c may be provided with a dedicated control valve 208. Alternatively, and as illustrated in FIG. 2 and in FIG. 10, all emission lines 207a, 207b, 207c may be controlled by a common control valve 222 (also referred to herein as the common emission valve). An emission switching module 217 may be provided to open or close the fluidic connection between each emission line 207a, 207b, 207c and the common control valve 222.

The pressure in each liquid reservoir 204a, 204b, 204c may be monitored by a respective pressure sensor 210 as described above. The gaseous and liquid media in each liquid reservoir 204a, 204b, 204c may mix together through the use of a respective agitating element 211. Liquid may flow from each liquid reservoir 204a, 204b, 204c via respective feeding lines 213a, 213b, 213c, sequentially or in parallel, towards the culture system 201. A feeding switching module 224 may direct the flow of liquid from one or more of the feeding lines 213a, 213b, 213c to the culture system 201. The feeding lines 213a, 213b, 213c, may join into a single feeding line to an inlet of the culture system 201 (as illustrated); or the feeding lines 213a, 213b, 213c may be independently connected to different inlets of the culture system 201.

A liquid flow sensor 221 may be positioned between an outlet of one of the liquid reservoirs 204a, 204b, 204c and an inlet of the culture system 201, e.g., between the feeding switching module 224 and an inlet of the culture system 201 to measure the flow rate of the liquid medium 205a, 205b and/or 205c flowing into the culture system 201. After flowing through the culture system 201, the liquid medium 205a, 205b and/or 205c may be collected via at least one collecting line 214 in a collecting reservoir 215 as described above. According to some embodiments, the collecting reservoir 215 may comprise a pressure controller for applying a counter pressure at the outlet of the culture system 201, as described above. An extra reservoir 216 may be connected in parallel to the collecting reservoir 215, so as to bypass the culture system 201. The feeding switching module 224 may orient the liquid medium from one or more of the reservoirs 204 (several inlets ports) to the culture system 201 and/or to the extra reservoir 216 (two outlet ports). One possible implementation of the feeding switching module 224 is a set of several (as illustrated: three) on/off valves connected to 3/2 valve.

The extra reservoir 216 may in particular be used for any draining or purging operations. In particular, in an initial step of the method, liquid medium may be flowed to the extra reservoir 216 in order to purge air from the system and to avoid sending air to the culture system 201.

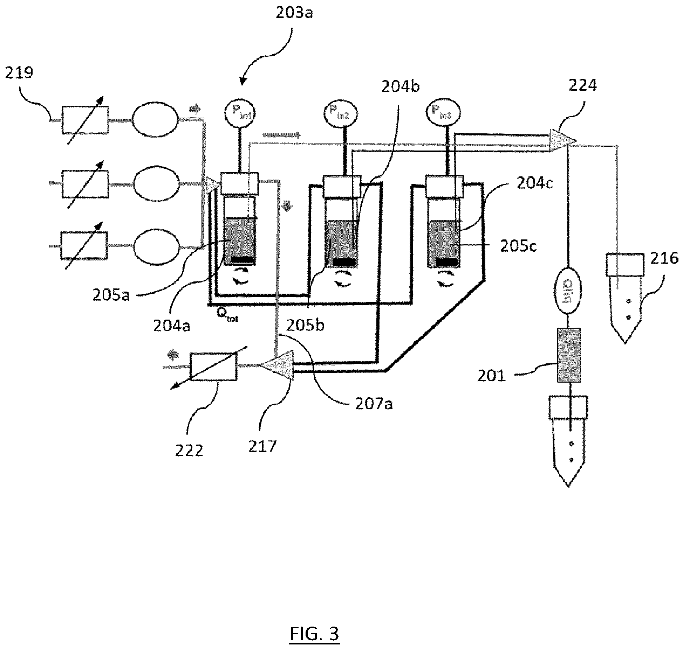

One step of the method of the invention implemented with this assembly is illustrated in FIG. 3. In this step, a combination of gases from three gas sources 219 (such as $N_2$, $CO_2$ and air) flows in a controlled manner into the liquid reservoir 204a of the first feeding module 203a, and exits *via* the emission line 207a (controlled by the control valve 217) and the common emission valve 222 (see the arrows on the drawing). The pressure applied in the liquid reservoir 204a makes the liquid medium 205 flow with a controlled gas concentration and a controlled flow rate. The feeding switching module 224 orients the liquid medium 205a either to the extra reservoir 216 or to the culture system 201.

In different steps of the methods, and by acting on the various switching modules, various liquid media may be provided at different times to the culture system 201 and/or to the extra reservoir 216.

A liquid medium may in fact, flow from any number of feeding modules 203 to the culture system 201, in sequence or in parallel. The various steps of injection from each liquid reservoir 204a, 204b, 204c may be repeated according to the first sequence, or may be repeated according to a new sequence.

For example, according to one sequence, the liquid medium 205a may flow from the first liquid reservoir 204a to the culture system 201 before the liquid medium 205b from the second reservoir 204b flows to the culture system 201 before the liquid medium 205c from the third reservoir 204c flows to the culture system 201. According to other sequences, the order of injection may be modified (e.g., second then third then first reservoirs; or third then first then second reservoirs; etc.).

According to another sequence, all three liquid media 205a, 205b, 205c may be simultaneously injected to the culture system 201 (in parallel via a single inlet as represented in Fig 3, or through different inlets).

This allows for a flexible method and enables a sequence of solutions to be tested on the same living entity (e.g. a sequence of metabolic inhibitors, a sequence of treatments and/or a sequence of energy sources such as glucose, glutamine and/or acetyl-CoA). Preferably, such a sequential method may be used to combine various inhibitors with chemotherapeutic drugs.

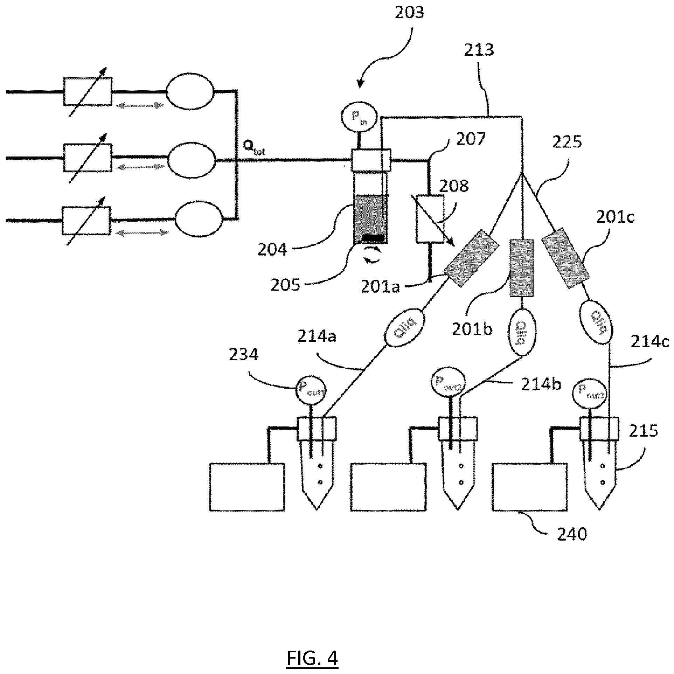

According to some embodiments and as shown in FIG. 4, a feeding device 203 may be fluidically connected to a plurality of culture systems 201a, 201b, 201c. Therefore, in the method of the invention, the liquid medium 205 may flow in sequence or in parallel from the at least one liquid reservoir 204 of the feeding device 203 to multiple culture systems 201a, 201b, 201c. In the illustrated embodiment, three culture systems 201a, 201b, 201c are present. Unless explicitly specified, the assembly and in particular the feeding device 203 and each culture system 201a, 201b, 201c may comprise all features described above in connection with the general principle of the invention.

The liquid medium 205 having a controlled dissolved gas concentration and a controlled flow rate may flow through a feeding line 213 fluidly connected to a feeding manifold 225 that supplies the liquid medium 205 to each available culture system 201a, 201b, 201c. Optionally, the feeding manifold 225 may be equipped with a switching module, so as to select the culture system(s) to which the liquid medium 205 flows, at each point in time. This enables the comparison of different living entity samples in real time and under exactly the same controlled conditions. The gaseous medium may be vented via a control valve 208 positioned on the emission line 207 at an outlet of the liquid reservoir 204. Each culture system 201a, 201b, 201c may be fluidically connected to at least one respective collecting line 214a, 214b, 214c at an outlet thereof. Each collecting line 214a, 214b, 214c may be fluidically connected to an inlet of a respective collecting reservoir 215. Pressure within the collecting reservoirs 215 may be monitored by respective pressure sensors 234, and controlled by respective pressure controllers 240.

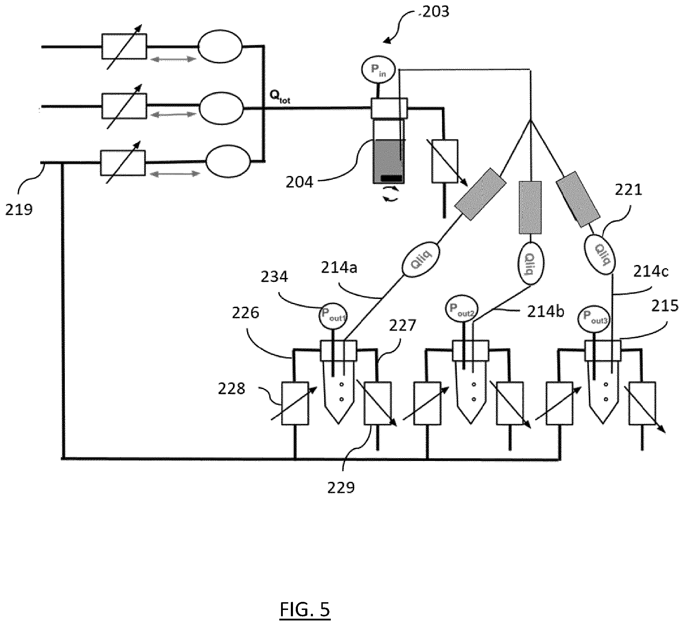

Alternatively and as presented in FIG. 5, instead of being controlled by a pressure controller 240, the pressure in each collecting reservoir 215 may be controlled similarly to the liquid reservoir 204. In other terms, each collecting reservoir 215 may be provided with an admission line 226 and an emission line 227. A control valve 228 may be provided on the admission line 226 and a control valve 229 may be provided on the as emission line 227. The admission line 226 may be fluidically connected to a gas source 219, which can be in particular the same gas source 219, or one of the gas sources 219, used for providing gaseous medium to the liquid reservoir 204 of the feeding device 203. The flow rate in each collecting line 214a, 214b, 214c may be monitored by individual liquid flow sensors 221.

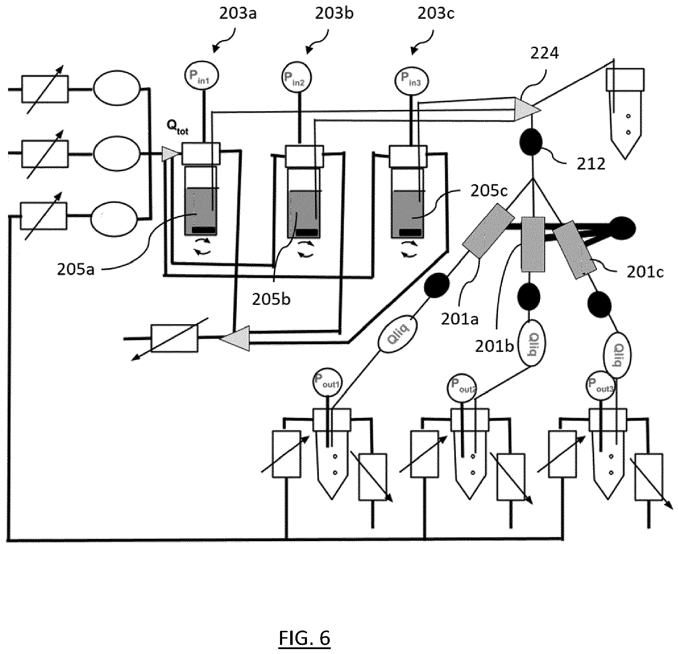

According to some embodiments and as shown in FIG. 6, the feeding device may comprise multiple feeding modules 203a, 203b, 203c and may be fluidically connected to multiple culture systems 201a, 201b, 201c. Therefore, in the method of the invention, different liquid media 205a, 205b, 205c may be provided in a sequential and/or parallel manner to various culture systems 201a, 201b, 201c. Unless explicitly specified, the assembly and in particular the feeding device 202 and each culture system 201a, 201b, 201c may comprise all features described above in connection with the general principle of the invention.

One or more switching modules 224 may be present to select liquid medium from one or more of the feeding modules 203a, 203b, 203c, and direct it to one or more of the culture systems 201a, 201b, 201c. In some variations, liquid medium from only one feeding module is simultaneously directed to all culture systems 201a, 201b, 201c. In other variations, liquid media from more than one feeding modules 203a, 203b, 203c are mixed and simultaneously directed to all culture systems 201a, 201b, 201c. In other variations liquid medium 205 (from only one feeding module, or mixed from more than one feeding module) is directed to only one or to a subset of the culture systems 201a, 201b, 201c.

This configuration enables any combination of solutions (e.g. a sequence of metabolic inhibitors, a sequence of treatments and/or a sequence of energy sources such as glucose, glutamine and/or acetyl-CoA) to be tested on a group of living entities in a controlled environment that closely represents the desired in vivo conditions. For example, a sequence of liquid media 205a, 205b, 205c may be provided to a group of living entity samples of the same batch, in order to achieve optimal accuracy in obtained results. Alternatively, a sequence of liquid media 205a, 205b, 205c may be provided to samples from different batches in order to carry out a comparative analysis on different living entities. Alternatively, different liquid media 205a, 205b, 205c may be provided to samples of the same or different batches. Alternatively, different sequences of the same liquid media 205a, 205b, 205c or different sequences of different liquid media 205a, 205b, 205c may be applied to samples of the same or different batches.

Measurements to determine the presence or concentration of at least one substance may be obtained for any of these aforementioned configurations through the use of sensors 212 positioned inside the culture systems 201a, 201b, 201c, upstream of the culture systems 201a, 201b, 201c, and/or downstream of the culture systems 201a, 201b, 201c.

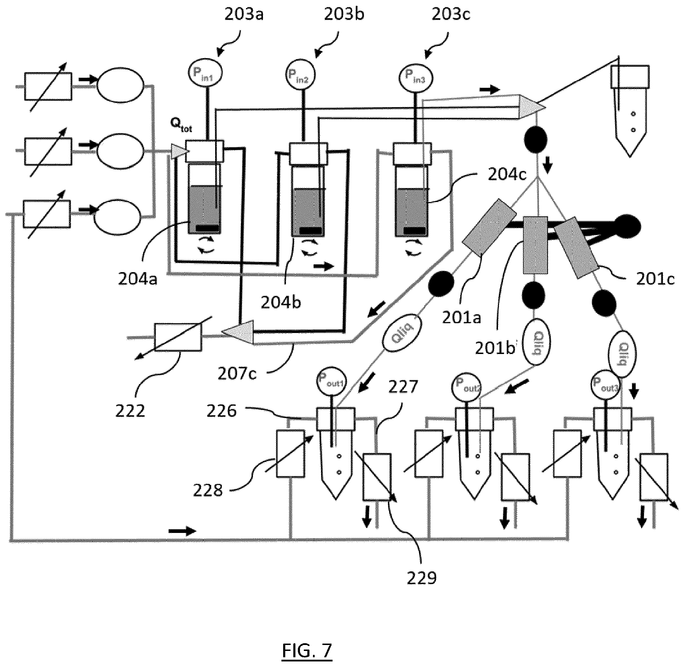

One step of the method of the invention implemented with this assembly is illustrated in FIG. 7. In this step, only one among three feeding modules 203a, 203b, 203c supplies three culture systems 201a, 201b, 201c in parallel (see the arrows). The gaseous medium from the liquid reservoir 204c of the active feeding module 203c is evacuated via the common control valve 222 on the emission line 207c. Counterpressure in the culture systems 201a, 201b, 201c is controlled by the control valves 228, 229 positioned on the admission and emission lines 226, 227 of the collecting reservoirs connected to each culture system 201a, 201b, 201c.

Preferably, such embodiments may be used to supply a sequence of energy sources (e.g. glucose, glutamine, and/or fatty acids) into a group of cell cultures 201a, 201b, 201c in order to simulate a process of anaplerosis (i.e. a fueling of the Krebs cycle), wherein a comparison of the oxygen consumption (OCR) is made for each energy source supplied to each cell culture 201a, 201b, 201c.

Preferably, such embodiments may also be used to inject a sequence of metabolic inhibitors (e.g. mitochondrial translation inhibitor) into culture systems 201a, 201b, 201c at controlled dissolved gas levels. FCCP, oligomycin, rotenone and antimycin A, carboxin, cyanide may be provided to culture systems 201a, 201b, 201c in order to measure the non-mitochondrial oxygen consumption.

According to some embodiments, a sequential supply of liquid medium to the culture system 201 may be used to rinse the system for cleaning purposes or to refresh the liquid medium 205. Therefore, the method may, for example, enable focusing on the effect of at least one specific compound extracellular concentration on the consumption or secretion of at least one compound.

According to some embodiments, a sequential supply of liquid medium 205 to the culture system 201 may be used to refill liquid reservoirs 204a, 204b, 204c with substances that were, for example, secreted by cells in the culture system 201, in order to observe the impact of the extracellular concentration.

According to some preferred embodiments, components secreted (for example by another cell type in another microfluidic chip/well) may also be injected into one or more of the liquid reservoirs 204a, 204b, 204c.

Recirculation

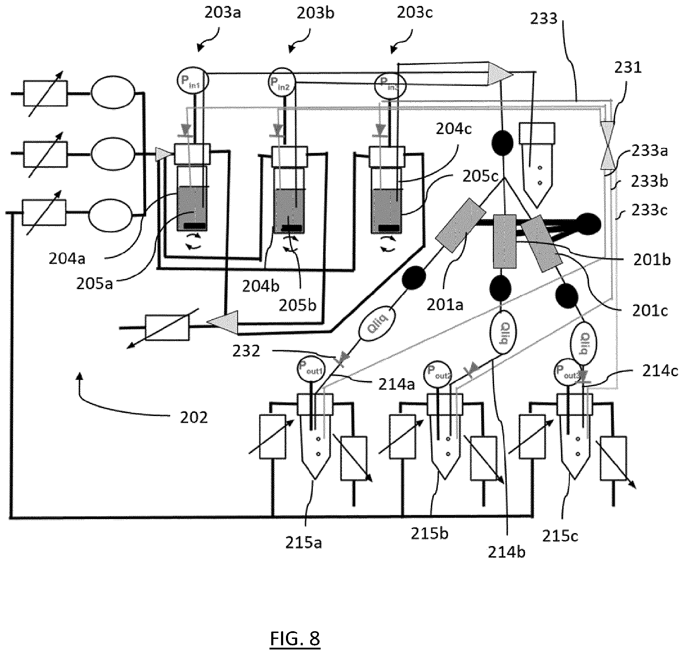

According to some embodiments, and as displayed in FIG. 8, the assembly may comprise a recirculation capability, i.e. it may comprise at least one liquid recirculation line 233 from a collecting reservoir 215 to a liquid reservoir in the feeding device 202. Thus, the method of the invention may comprise a step of withdrawing liquid medium 205a, 205b, 205c from the collecting reservoir 215 and flowing it back to the liquid reservoir 204a, 204b, 204c. Therefore, the liquid reservoir 204a, 204b, 204c may be refilled with liquid from the collecting reservoir 215. This may be carried out by applying a differential pressure between the collecting reservoir 215 and the liquid reservoir 204a, 204b, 204c.

When a plurality of collecting reservoirs 215a, 215b, 215c are present, each of these may be connected to such a recirculation line 233. When a plurality of feeding modules 203a, 203b, 203c and liquid reservoirs 204a, 204b, 204c are present, the one or more recirculation lines 203a, 203b, 203c may be connected to only one of the liquid reservoirs 204a, 204b, 204c, or to several or all of them. Each collecting reservoir 215a, 215b, 215c may be fluidically connected to one respective liquid reservoir 204a, 204b, 204c, or may be connected to several or all liquid reservoirs 204a, 204b, 204c. A recirculation switching module 231 may be present to connect various branches of the recirculation lines 233 together and to switch the recirculation pattern between various configurations. The recirculation switching module 231 may for example be implemented with a plurality (as illustrated: three) of on/off valves.

By way of example, in one configuration, liquid medium 205a, 205b, 205c may flow from all collecting reservoirs 215a, 215b, 215c to one liquid reservoir 204a, 204b, 204c in the feeding device 202, while, in another configuration, liquid medium 205a, 205b, 205c may flow from all collecting reservoirs 215a, 215b, 215c to one other liquid reservoir 204a, 204b, 204c in the feeding device 202.

By *"perfusing"* is meant flowing liquid medium 205 from the feeding device 202 to a culture system 201 and then preferably a collecting reservoir 214. By *"refilling"* is meant flowing liquid medium 205 from the collecting reservoir 215 to the feeding device 202.

The method of the invention may comprise one or more perfusing steps alternating with one or more refilling steps. In some cases, a perfusing step may be carried out simultaneously with a refilling step. For instance, a perfusing step may be conducted in connection with one culture system 201a, while a refilling step may be conducted in connection with a collecting reservoir associated with a different culture system 201b.

As illustrated in FIG. 8, each collecting reservoir 215a, 215b, 215c may be fluidically connected to each liquid reservoir 204a, 204b, 204c in the feeding device 202 through recirculation lines 233, and a recirculation switching module 231 may be present to direct liquid medium 205 from any of the collecting reservoirs 215a, 215b, 215c to one or more of the liquid reservoirs 204a, 204b, 204c in the feeding device 202.

In order to avoid any undesired backflow, a one-way valve 232 such as a check valve may be present on each collecting line 214a, 214b, 214c. Similarly, a one-way valve 232 such as a check valve may be present on each recirculation line 233a, 233b, 233c.

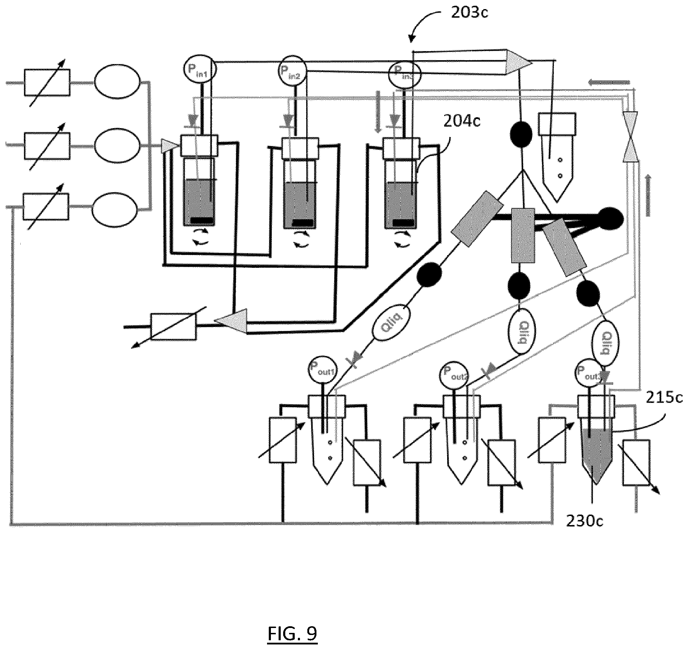

One refilling step of the method of the invention implemented with this assembly is illustrated in FIG. 9. In this step, liquid medium 230c from one of the collecting reservoirs 215c is recovered back to the liquid reservoir 204c of one of the feeding modules 203c (see the arrows).

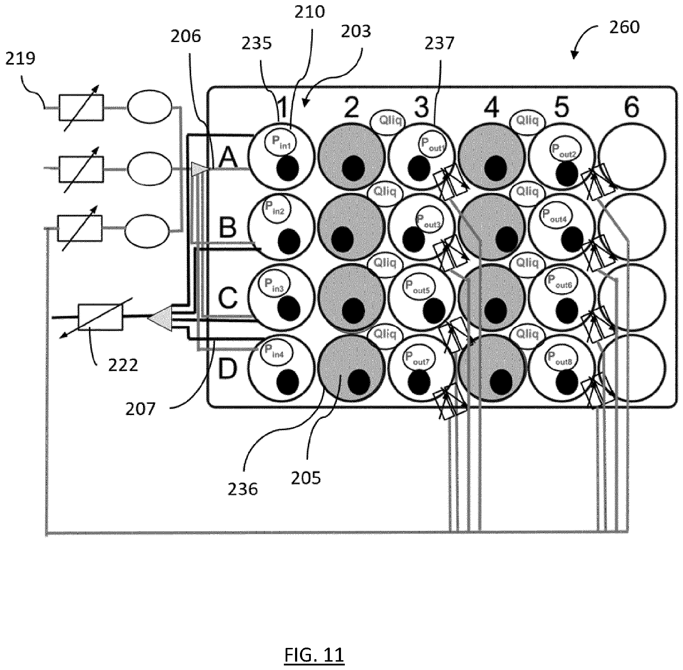

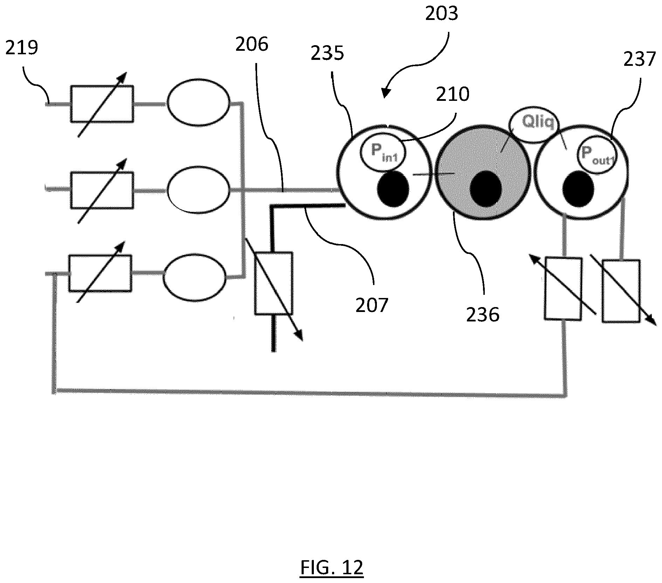

According to some embodiments, and as shown in FIG. 11 and FIG. 12, the assembly may comprise a well plate 260. The well plate 260 may be made of various materials such as low gas-permeable plastic, or preferably glass. Fluidic connections (e.g., microfluidic channels) may be provided between some of the wells 235, 236, 237, in particular adjacent wells 235, 236, 237. A plurality of feeding modules 203 may be formed by respective groups of wells 235, 237 in the well plate 260. This integrated system allows the monitoring of multiple replicates within a single experimental microplate.

For example, one or more gas sources 219 may be connected (as described above) via respective admission lines 206 to a first series of wells 235 (for example one or more rows or columns in the well plate 260) equipped with one or more sensors, such as a pressure sensor 210: see column 1 in FIG. 11. These wells 235 may also be associated with respective emission lines 207, which may for instance be controlled by a common control valve 222. Each of these pressurized wells 235 in the first series may be fluidically connected to a second series of wells 236 (for example one or more rows or columns in the well plate 260) containing liquid medium 205, i.e. liquid reservoirs 236: see columns 2 and 4 in FIG. 11. The pressure set in the wells 235 of the first series may thus be applied to the wells 236 of the second series. Each of these liquid reservoirs 236 in the second series of wells 236 may be fluidically connected to respective culture systems separate from the well plate, via respective feeding lines, and then to a third series of wells 237 (for example one or more rows or columns in the well plate 260) acting as collecting reservoirs 237, via respective collecting lines: see columns 3 and 5 in FIG. 11. Each of the collecting reservoirs 237 may be pressurized owing to a fluidic connection with a gas source 219, which may be for example one of the gas sources 219 providing gaseous medium to the first series of wells 235.

In some embodiments, each well 236 of the second series is associated with a single well 237 in the third series. Each well 235 of the first series may be associated with a single well 236 in the second series, or, as illustrated in FIG. 11, to more than one wells 236 in the second series.

Alternatively, part or all of the reservoirs may be provided in another module distinct from the well plate and fluidically connected to the well plate.

Variations of implementation of the method

Some possible sequences of steps in the method of the invention are disclosed herein. These sequences of steps may be implemented in some or all of the assemblies describe above.

In some variations, the method may comprise a first step of supplying liquid medium having a concentration of dissolved oxygen of less than 18.6%, preferably of 10% or less, more preferably of 5% or less, to the culture system, so as to incubate the living entity (e.g. cells) in the culture system in physioxic or hypoxic conditions, a second step of supplying a test compound such as a drug (e.g., a chemotherapy drug) to the culture system via the liquid medium, followed by a third step of rinsing the culture system by supplying liquid medium without the test compound to the culture system.

In some variations, the method may comprise taking at least one measurement wherein the liquid medium fed to the culture system has a concentration of dissolved gas, preferably oxygen, at a first value; and at least one measurement wherein the liquid medium fed to the culture system has a concentration of dissolved gas, preferably oxygen, at a second value different from the first value.

In some variations, the method may comprise firstly feeding liquid medium to the culture system having a concentration of dissolved gas, preferably oxygen, at a first value; and then secondly feeding liquid medium to the culture system having a concentration of dissolved gas, preferably oxygen, at a second value different from the first value and taking at least one measurement. This makes it possible to incubate the living entity (e.g. cells) in the culture system in certain conditions (such as hypoxic conditions) and then testing the living entity in other conditions.

In some variations, the method may comprise taking at least one measurement wherein the liquid medium fed to the culture system has a concentration of dissolved gas, preferably oxygen, at a first value; and at least one measurement wherein the liquid medium fed to the culture system has a concentration of dissolved gas, preferably oxygen, at a second value different from the first value. This makes it possible to successively test the living entity for example in physioxic conditions and hypoxic conditions.

In some variations, the method may comprise taking at least one measurement wherein the liquid medium fed to the culture system has a concentration of a nutrient or metabolite, preferably glucose, at a first value; and at least one measurement wherein the liquid medium fed to the culture system has a concentration of the nutrient or metabolite, preferably glucose, at a second value different from the first value.

The above modalities may be combined, i.e. the concentration of at least two species may be varied independently over time or in successive steps, while taking measurements, so as to monitor the influence of each species on the production or consumption of at least one substance.

In some variations, the method may include collecting liquid medium from a first culture system and feeding it to a liquid reservoir prior to flowing the liquid medium from said liquid reservoir to a second culture system. Both culture systems may preferably be part of the same assembly. This makes it possible to feed substances secreted by a living entity to another type of living entity or to the same type living entity cultured in different conditions. For instance, the living entity from which the secreted substances are collected can be tumoral cells and the other type of living entity to which the secreted substances are supplied can be non-tumoral cells, or conversely.

In some variations, liquid medium from a liquid reservoir having a first dissolved gas (e.g. oxygen) concentration may be supplied to a culture system from a liquid reservoir as a first step; liquid medium (including for example secreted substances) may then be collected to refill the same liquid reservoir (or another liquid reservoir) before being fed as a second step to another culture system, or to the same culture system, with a second dissolved gas (e.g. oxygen) concentration different from the first concentration. For example, the second oxygen concentration may be higher than the first oxygen concentration. In this case, substances secreted in hypoxic conditions may be fed to a culture system in normoxic or physioxic conditions, as defined above. Or the second oxygen concentration may be lower than the first oxygen concentration. In this case, substances secreted in normoxic or physioxic conditions may be fed to a culture system in hypoxic conditions, as defined above.

In some variations, a first liquid medium which is a growth medium may be injected into a culture system to cultivate the living entity (preferably cells) over a period of time of e.g. 1 hour to 7 days, such as approximately 1 day, before injecting a second liquid medium different from the first liquid medium and which is a treatment medium comprising at least one drug (e.g. a chemotherapy or immunotherapy drug) into the cell culture system over a period of time of e.g. 1 minute to 1 month, preferably 1 hour to 7 days, such as approximately 2 days. Optionally, as a third step, a third liquid medium which is a growth medium (and which can be the same as the first liquid medium) may be provided again to the culture system.

In some variations, a sequential supply of liquid medium to the culture system may be used to stimulate cells using shear stress. This enables monitoring of how the stimulation may modify parameters such as, for example, their metabolism and/or their extracellular vesicles (EVs) secretion. The liquid medium may, for example, be supplied at a high flow rate to a culture system. Secreted components may then be collected and sent into a different culture system preferably in the same assembly, possibly at a different, lower flow rate. This may be useful in particular to instigate vesicle secretion in a first step and then feed the obtained vesicles to another culture system.

The method according to the invention may be implemented so as to take measurements relating to cell secretion and/or consumption. The method according to the invention may also be implemented to perform biochemical reactions, or enzymatic reactions. In addition, according to some preferred embodiments, the method according to the invention is used for a drug screening operation, or a toxicity screening operation.

In some variations, the method of the invention may be used to test drugs promoting ROS increase. Additionally or alternatively, the method may be used to test drugs targeting metabolic properties of cells by combining metabolic inhibitors in order to increase cell death and overcome drug resistance. As the system enables sequential injection and combination of different liquid media, and determining presence or concentration of a substance, the combination of different inhibitors with chemotherapeutic drugs may be tested in a controlled environment.

EXAMPLES

A test was executed using an assembly as shown in FIG. 1, using a commercial chip from BeOneChip (BeFlow) as a culture system 201. A liquid medium 205 was supplied to the culture system 201 at a flow rate of 20 $\mu$L/min (corresponding to a shear stress of $6.23 \times 10^{-1}$ dyn/cm$^2$) and at different partial pressures of oxygen (initially 140 mmHg, and then slow decrease of oxygen by decreasing the speed of equilibration in the reservoir). A Nanoparticle Tracking Analysis system, upstream and downstream of the culture system 201 was used to measure the number of extracellular vesicles in the cell culture medium 205 The method enabled the determination an average of $3 \times 10^3$ extracellular vesicles per cell (EVs/cell) over a period of 2 hours.

In this test, it was possible to independently investigate the effects of the flow rate (and hence shear stress) and partial pressure of oxygen on the number of extracellular vesicles secreted.

**Claims**

1. A method for monitoring at least one substance which may be produced or consumed by at least one living entity, the method comprising:

   - providing a culture system (201) comprising said living entity;
   - providing a feeding device (202) fluidically connected to the culture system (201), the feeding device (202) comprising at least one feeding module (203), and each feeding module (203) comprising:

- a liquid reservoir (204) containing a liquid medium (205) and provided with a gas inlet, a gas outlet and a liquid outlet,
- an admission line (206) configured to provide a gaseous medium into the liquid reservoir (204), the admission line (206) being connected to the gas inlet, and
- an emission line (207) configured to withdraw the gaseous medium from the liquid reservoir (204), the emission line (207) being connected to the gas outlet;

the feeding device (202) further comprising at least one control valve (218) associated with the admission line(s) (206) and at least one control valve (208) associated with the emission line(s) (207);
- flowing gaseous medium into the at least one liquid reservoir (204) via the admission line (206) and out of the at least one liquid reservoir (204) via the emission line (207), so as to apply a pressure in the at least one liquid reservoir (204);
- flowing the liquid medium (205) with a controlled concentration of a dissolved gas and at a controlled flow rate, from the at least one liquid reservoir (204) to the culture system (201) due to the pressure in the at least one liquid reservoir (204);

the method further comprising:

- taking at least one measurement within the culture system (201) so as to determine the presence, concentration or amount of the at least one substance in the liquid medium (205) in the culture system (201); and/or
- taking at least a first measurement upstream of the culture system (201) and a second measurement downstream of the culture system (201), and determining a concentration or amount of the substance consumed or produced by the at least one living entity based on the difference between the second measurement and the first measurement.

2. The method according to claim 1, comprising taking multiple successive measurements so as to determine the evolution over time of the presence or concentration of the at least one substance.

3. The method according to claim 1 or 2, wherein the at least one substance is a nutrient or metabolite, preferably selected from lactate, glucose and/or an amino acid; and wherein, preferably, the measurement comprises the direct measurement of the at least one substance.

4. The method according to any one of claims 1 to 3, wherein the feeding device comprises at least two feeding modules, comprising different liquid media, the method comprising a step of flowing liquid medium from the liquid reservoir of a first feeding module to the culture system, and then flowing liquid medium from the liquid reservoir of a second feeding module to the culture system.

5. The method according to any one of claims 1 to 4, comprising a step of flowing liquid medium in sequence or in parallel from the at least one liquid reservoir to multiple culture systems.

6. The method according to any one of claims 1 to 5, comprising a step of collecting liquid medium from the culture system and refilling at least one liquid reservoir (204) of the feeding device (202) with the collected liquid medium.

7. The method according to any one of claims 1 to 6, comprising a step of controlling the control valve(s) associated with the admission line(s) and/or the control valve(s) associated with the emission line(s) to adjust at least one concentration of dissolved gas, preferably oxygen and/or carbon dioxide, in the liquid medium to a predetermined value, preferably using a closed loop regulation and/or the method comprising a step of controlling the control valve(s) associated with the admission line(s) and/or the control valve(s) associated with the emission line(s) to adjust the pH of the liquid medium to a predetermined value, preferably using a closed loop regulation and/or the method comprising a step of controlling a counter-pressure at an outlet of the culture system to adjust the flow rate of the liquid medium from the liquid reservoir to the culture system, preferably using a closed loop regulation, or the method comprising controlling a hydraulic resistance downstream of the liquid reservoir to adjust the flow rate of the liquid medium from the liquid reservoir to the culture system, preferably using a closed loop regulation.

8. The method according to any one of claims 1 to 7, wherein liquid medium flowed to the culture system has a concentration of dissolved oxygen of approximately 18.6% or less, preferably of approximately 10% or less, more preferably of approximately 5% or less.

9. The method according to any one of claims 1 to 8, wherein the liquid medium flowed to the culture system contains one or more components selected from nutrients, drugs such as chemotherapy drugs, immunotherapy drugs, hormonotherapy drugs and inhibitors.

10. The method according to any one of claims 1 to 9, comprising taking at least one measurement wherein the liquid medium has a concentration of dissolved gas, preferably oxygen, at a first value; and at least one measurement wherein the liquid medium has a concentration of dissolved gas, preferably oxygen, at a second value different from the first value.

11. The method according to any one of claims 1 to 10, comprising collecting liquid medium from another culture system and feeding it to the at least one liquid reservoir (204) prior to flowing the liquid medium (206) from said liquid reservoir (204) to the culture system (201).

12. The method according to any one of claims 1 to 11, comprising an incubation step wherein liquid medium devoid of a test compound is flowed to the culture system, followed by a test step wherein liquid medium comprising a test compound is flowed to the culture system, optionally followed by a rinsing step wherein liquid medium devoid of the test compound is flowed to the culture system.

13. An assembly, comprising:

- a culture system (201);
- a feeding device (202) fluidically connected to the culture system (201), the feeding device (202) comprising at least one feeding module (203), and each feeding module (203) comprising:

- a liquid reservoir (204) containing a liquid medium (205) and provided with a gas inlet, a gas outlet and a liquid outlet,
- an admission line (206) configured to provide a gaseous medium into the liquid reservoir (204), the admission line (206) being connected to the gas inlet, and
- an emission line (207) configured to withdraw the gaseous medium from the liquid reservoir (204), the emission line (207) being connected to the gas outlet;

the feeding device (202) further comprising at least one control valve (218) associated with the admission line(s) (206) and at least one control valve (208) associated with the emission line(s) (207);
- at least one sensor (212) for measuring the presence or concentration of at least one substance in the liquid medium (205), positioned inside the culture system (201), and/or at least two sensors (212) for measuring the presence or concentration of at least one substance in the liquid medium (205), placed upstream of the culture system (201) and downstream of the culture system (201).

14. The assembly according to claim 13, wherein the at least one culture system is a cell culture system, preferably selected from a microfluidic cell culture system, a millifluidic cell culture system or a nanofluidic cell culture system.

15. The assembly according to claim 13 or 14, wherein the sensor(s) comprise at least one metabolic sensor, preferably selected from glucose sensors, lactate sensors and/or amino acid sensors; and/or the sensor(s) comprise at least one pH sensor and/or oxygen sensor.

EP 4 245 839 A1

FIG. 1

FIG. 2

FIG. 3

FIG. 4

FIG. 5

FIG. 6

FIG. 7

FIG. 8

FIG. 9

FIG. 10

FIG. 11

FIG. 12

Europäisches
Patentamt
European
Patent Office
Office européen
des brevets

# EUROPEAN SEARCH REPORT

**Application Number**

EP 22 30 5292

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/010861 A1 (HARVARD COLLEGE [US]; HINOJOSA CHRISTOPHER DAVID [US] ET AL.) 21 January 2016 (2016-01-21) * claims 1,2 * | 1-15 | INV. C12M1/34 C12M1/00 |
| A | WO 2008/136339 A1 (TOYO SEIKAN KAISHA LTD [JP]; TANAKA SATOSHI [JP] ET AL.) 13 November 2008 (2008-11-13) * claim 1 * | 1-15 | |
| A | WO 2019/191685 A1 (UNIV ARIZONA [US]) 3 October 2019 (2019-10-03) * claim 1 * | 1-15 | |
| A | RU 2 587 628 C1 (OBSHSHESTVO S OGRANICHENNOJ OTVETSTVENNOSTYU NT TS BIOKLINIKUM [RU]) 20 June 2016 (2016-06-20) * claim 1 * | 1-15 | |
| A | US 2017/198246 A1 (NIAZI SARFARAZ K [US]) 13 July 2017 (2017-07-13) * claim 1 * | 1-15 | TECHNICAL FIELDS SEARCHED (IPC) C12M |

The present search report has been drawn up for all claims

| Place of search | Date of completion of the search | Examiner |
|---|---|---|
| Berlin | 12 September 2022 | Jones, Laura |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 30 5292

12-09-2022

| Patent document cited in search report | | Publication date | Patent family member(s) | | Publication date |
|---|---|---|---|---|---|
| WO 2016010861 | A1 | 21-01-2016 | AU 2015289999 | A1 | 09-03-2017 |
| | | | CA 2955172 | A1 | 21-01-2016 |
| | | | EP 3169626 | A1 | 24-05-2017 |
| | | | GB 2546424 | A | 19-07-2017 |
| | | | JP 6815985 | B2 | 20-01-2021 |
| | | | JP 6931415 | B2 | 01-09-2021 |
| | | | JP 2017525345 | A | 07-09-2017 |
| | | | JP 2021073999 | A | 20-05-2021 |
| | | | US 2017121658 | A1 | 04-05-2017 |
| | | | US 2017121659 | A1 | 04-05-2017 |
| | | | US 2017121663 | A1 | 04-05-2017 |
| | | | WO 2016010861 | A1 | 21-01-2016 |
| WO 2008136339 | A1 | 13-11-2008 | CN 101668844 | A | 10-03-2010 |
| | | | EP 2141226 | A1 | 06-01-2010 |
| | | | JP 5023795 | B2 | 12-09-2012 |
| | | | JP 2008271850 | A | 13-11-2008 |
| | | | KR 20100015668 | A | 12-02-2010 |
| | | | US 2010062530 | A1 | 11-03-2010 |
| | | | WO 2008136339 | A1 | 13-11-2008 |
| WO 2019191685 | A1 | 03-10-2019 | US 2021079337 | A1 | 18-03-2021 |
| | | | WO 2019191685 | A1 | 03-10-2019 |
| RU 2587628 | C1 | 20-06-2016 | NONE | | |
| US 2017198246 | A1 | 13-07-2017 | NONE | | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107430055 A
- CN 110669671 A
- CN 104428672 A
- WO 2008024855 A1
- KR 20160149109 A
- FR 3075823